# EUROPEAN PATENT APPLICATION

(11) **EP 2 242 335 A1**
(43) Date of publication of application: **20.10.2010**
(21) Application number: 08855844.0
(22) Date of filing: 27.11.2008
(51) Int. Cl.: H05B 37/02, F21S 8/02, F21S 8/04, F21Y 101/02

(54) **LIGHTING APPARATUS**

(30) Priority: 07.12.2007 JP 2007317501
(71) Applicant: Sharp Kabushiki Kaisha, Osaka-shi Osaka 545-8522 (JP)
(72) Inventor: OYAMA, Kazuma, Osaka-shi, Osaka 545-8522 (JP)
(74) Representative: Treeby, Philip David William
(86) International application number: PCT/JP2008/071529
(87) International publication number: WO 2009/072430

(57) **Abstract**

There is provided a lighting apparatus capable of regulating the amount of light in a wavelength range inhibiting melatonin secretion which is emitted from a luminous portion in a direction toward eyes of a user to change an influence on the inhibition of melatonin secretion, and performing adequate lighting in accordance with time or a lifestyle of the user. The lighting apparatus is configured such that first luminous portions 3, 3 ··· and second luminous portions 4 and 4 having different light distribution characteristics are used, and respective luminous intensities of the first luminous portions 3, 3 ··· and the second luminous portions 4 and 4 are controlled by a control portion to change the amount of light in the wavelength range inhibiting melatonin secretion which is emitted in a predetermined direction from the first luminous portions 3, 3 ··· and the second luminous portions 4 and 4. As a result, by changing the amount of light emitted in the direction (predetermined direction) toward the eyes of the user in a normal usage mode, it is possible to regulate the amount of the light in the wavelength range inhibiting melatonin secretion which enters the eyes of the user to change the influence on the inhibition of melatonin secretion, and also perform adequate lighting in accordance with time or the lifestyle of the user.

## Description

### [Technical Field]

The present invention relates to a lighting apparatus which regulates the amount of light in a wavelength range inhibiting melatonin secretion which is emitted in a predetermined direction from a luminous portion.

### [Background Art]

In recent years, there is used a lighting apparatus including a luminous portion having various light sources such as an incandescent lamp, a low-pressure discharge fluorescent lamp, a fluorescent lamp, a light-emitting diode (hereinbelow referred to as an LED), and the like.

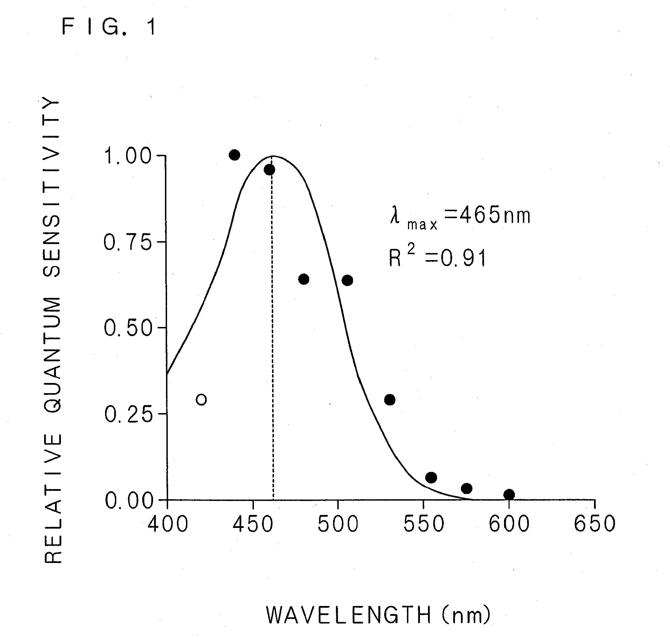

It is known that, by illuminating a room in a house using a light source which emits light having a low color temperature such as that of a light bulb color at night, in particular, before bedtime, it is possible to encourage smooth initiation of sleep when compared with a case where the room is illuminated using a light source which emits light having a high color temperature such as that of a daylight white color, a daylight color, or the like. This is because the secretion of melatonin, which is a hormone secreted in a brain and is considered to encourage a reduction in body temperature and initiation of sleep, is influenced by a color temperature of light entering the eyes of a human, i.e., a wavelength of light. With regard to the influence on an inhibition of melatonin secretion by the wavelength of light, consequences as shown in FIG. 1 are obtained by a study conducted by Brenard et al., and it can be seen from the consequences that a sky-blue wavelength range in the vicinity of 464 nm is influential in inhibiting the melatonin secretion.

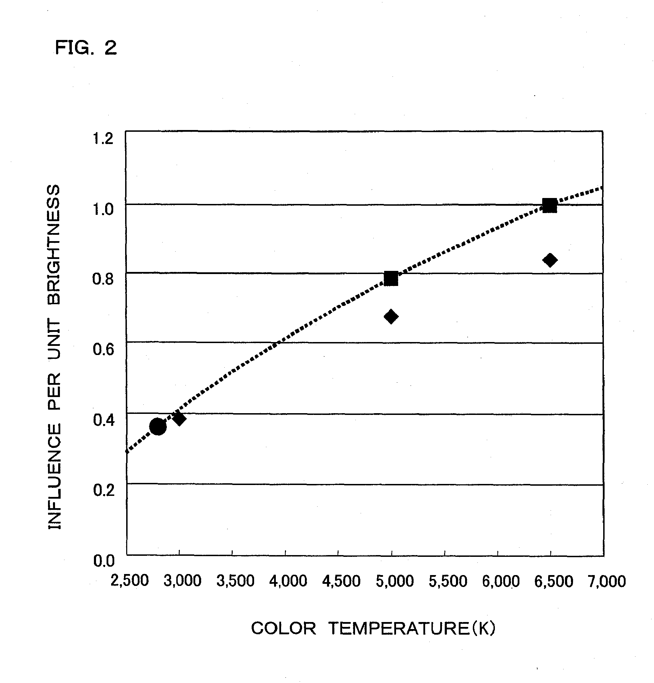

FIG. 2 shows a comparison of influences on the inhibition of melatonin secretion per unit brightness. The horizontal axis of FIG. 2 indicates a color temperature (K), while the vertical axis thereof indicates an influence on the inhibition of melatonin secretion per unit brightness. In the drawing, influences on the inhibition of melatonin secretion in standard light sources are indicated by a broken line, an influence in a light bulb having a color temperature of 2800 K (black body radiation) is indicated by a filled-in circle, and influences in the standard light sources having color temperatures of 5000 K (black body radiation) and 6500 K (D65 standard light source) are indicated by filled-in squares. In addition, in the drawing, influences on the inhibition of melatonin secretion in fluorescent lamps having color temperatures of 3000 K, 5000 K, and 6500 K are indicated by filled-in rhombuses. It is to be noted that the vertical axis shows relative values when a melatonin inhibition light intensity (influence on the inhibition of melatonin secretion) at 6500 K (D65 standard light source) is assumed to be 1, and the influence on the inhibition of melatonin secretion is larger as the value is higher. In a lighting apparatus including a plurality of light sources having different color temperatures, when the color of the light source to be turned on is changed from the daylight color to the light bulb color, it can be seen that the influence on the inhibition of melatonin secretion is reduced to be about one-third.

By the way, in general, a lighting apparatus is preferably configured such that light entering the eyes of a user does not include a wavelength range in the vicinity of 464 nm in order to encourage smooth initiation of sleep at night, while in the morning, the light entering the eyes of a user includes the wavelength range in the vicinity of 464 nm in order to encourage smooth awakening.

As such a lighting apparatus, there can be considered a lighting apparatus configured such that the influence on the inhibition of melatonin secretion is changed by switching between a plurality of light sources having different color temperatures such as those of the daylight color and the light bulb color, as described above, i.e., by turning on the light source having the light bulb color at night, and turning on the light source having the daylight color in the morning in a switching manner. However, the lighting apparatus has a problem that the color temperature can not be changed in accordance with a preference of a user.

A lighting apparatus disclosed in Patent Document 1 is configured such that a narrow-angle light source for illuminating only an area where a target object to be seen is placed, and an ambient illumination light source for illuminating a wide area including the area mentioned above are provided, and the ambient illumination light source is normally turned on, while the narrow-angle light source is turned on during reading.
[Patent document 1] Japanese Patent Application Laid-Open No. 11-273420

### [Disclosure of the Invention]

### [Problem to be Solved by the Invention]

However, in the lighting apparatus disclosed in Patent Document 1, when the narrow-angle light source is turned on, a narrow area immediately below the light source is brightly illuminated, but a peripheral area is dimly illuminated. Accordingly, it is possible to suppress the amount of light entering the eyes of a user, while an illuminance in the entire room is not sufficient. On the other hand, when the peripheral illumination light source is turned on, it is possible to illuminate the entire room brightly, but its light is emitted in many directions so that it is not possible to suppress the amount of light entering the eyes of the user. Thus, the lighting apparatus disclosed in Patent Document 1 has a problem that it is not possible to adequately perform lighting diversified in accordance with time or a lifestyle of the user.

The present invention has been achieved in view of such circumstances, and an object thereof is to provide a lighting apparatus capable of regulating the amount of light in a wavelength range inhibiting melatonin secretion which is emitted from a luminous portion in a direction toward eyes of a user to change an influence on the inhibition of melatonin secretion, and performing adequate lighting in accordance with time or a lifestyle of the user.

### [Means for Solving the Problem]

A lighting apparatus according to the present invention includes a plurality of luminous portions, and control means for controlling turning-on of the plurality of luminous portions, wherein the control means controls at least one of a luminous intensity and a color temperature of each of the plurality of luminous portions to regulate an amount of light in a wavelength range inhibiting melatonin secretion which is emitted in a predetermined direction.

In the present invention, by controlling at least one of the luminous intensity and the color temperature of each of the plurality of luminous portions, it is possible to change the amount of the light in the wavelength range inhibiting melatonin secretion which is emitted in a direction (predetermined direction) toward eyes of a user in a normal usage mode, regulate the amount of the light in the wavelength range which enters the eyes of the user to change an influence on the inhibition of melatonin secretion, and also perform adequate lighting in accordance with time or a lifestyle of the user.

In the lighting apparatus according to the present invention, each of the plurality of luminous portions has a different directivity, and at least one of the plurality of luminous portions has a light source, and a shield which shields light emitted in a predetermined direction from the light source.

In the present invention, the plurality of luminous portions having different directivities are used, and at least one of the plurality of luminous portions has the light source, and the shield for shielding the light emitted in the predetermined direction from the light source. By forming the shield properly, it is possible to obtain a sufficient illuminance on an illuminated surface illuminated with the light from the luminous portion, e.g., on a floor surface, while shielding the light emitted in the direction (predetermined direction) toward the eyes of the user from the luminous portion in a normal usage mode. By performing turning-on control of the plurality of luminous portions properly to change the amount of the light emitted in the direction toward the eyes of the user in the normal usage mode, it is possible to regulate the amount of the light in the wavelength range inhibiting melatonin secretion which enters the eyes of the user to change the influence on the inhibition of melatonin secretion, and also perform adequate lighting in accordance with time or the lifestyle of the user.

The lighting apparatus according to the present invention is attachable to a ceiling, and changes an amount of light emitted in a substantially horizontal direction from the luminous portion.

In the present invention, in the lighting apparatus which is attached to the ceiling, since the lighting apparatus is configured to change the amount of the light emitted from the luminous portion in a substantially horizontal direction, it is possible to change the amount of the light emitted in the direction toward the eyes of the user in the normal usage mode, and regulate the amount of the light in the wavelength range inhibiting melatonin secretion which enters the eyes of the user from the luminous portion to change the influence on the inhibition of melatonin secretion.

In the lighting apparatus according to the present invention, the control means controls the luminous intensity of the luminous portion to change an illuminance on an illuminated surface illuminated with light from the luminous portion.

In the present invention, since the illuminance is changed by controlling the luminous intensity of the luminous portion, it is possible to further change the influence on the inhibition of melatonin secretion by changing the absolute amount of the light, and also perform more adequate lighting in accordance with time or the lifestyle of the user.

In the lighting apparatus according to the present invention, the luminous portion includes a plurality of light sources having different luminous colors, and the control means controls the luminous intensity of each of the plurality of light sources to change the color temperature on an illuminated surface illuminated with light from the plurality of light sources.

In the present invention, the plurality of light sources having different luminous colors are used, and the color temperature on the illuminated surface is changed by controlling the respective luminous intensities of the plurality of light sources. By selecting the light sources properly, and setting the luminous intensities properly, it is possible to further change the influence on the inhibition of melatonin secretion, and also perform more adequate lighting in accordance with time or the lifestyle of the user.

In the lighting apparatus according to the present invention, the luminous portion has a plurality of light sources including a red light source, a green light source, and a plurality of blue light sources having different peak wavelengths, and the control means turns on the the plurality of blue light sources in a switching manner.

In the present invention, the plurality of light sources including the red light source, the green light source, and the plurality of blue light sources having different peak wavelengths are used, and the plurality of blue light sources are turned on in a switching manner. By selecting the plurality of blue light sources properly, e.g., by using the blue light source which emits light with the peak wavelength in the wavelength range in the vicinity of 464 nm, and the blue light source which emits light without the peak wavelength in the wavelength range in the vicinity of 464 nm, it is possible to further change the influence on the inhibition of melatonin secretion, and also perform more adequate lighting in accordance with time or the lifestyle of the user.

In the lighting apparatus according to the present invention, the control means includes a clock portion, and controls the luminous intensity of the luminous portion in accordance with time of the clock portion.

In the present invention, since the luminous intensity of the luminous portion is controlled according to time, it is possible to automatically change the influence on the inhibition of melatonin secretion without setting by the user, and also perform adequate lighting in accordance with time.

The lighting apparatus according to the present invention includes an illuminance sensor for detecting the illuminance, wherein the control means controls the luminous intensity of the luminous portion in accordance with the illuminance detected by the illuminance sensor.

In the present invention, since the luminous intensity of the luminous portion is controlled in accordance with the illuminance detected by the illuminance sensor, it is possible to perform adequate lighting, and also contribute to energy conservation.

### [Effect of the Invention]

According to the present invention, it is possible to regulate the amount of light in a wavelength range inhibiting melatonin secretion which is emitted from a luminous portion in a direction toward eyes of a user to change an influence on an inhibition of melatonin secretion, and perform adequate lighting in accordance with time or a lifestyle of the user.

### [Brief Description of the Drawings]

FIG. 1 is a wavelength characteristic view of an inhibition of melatonin secretion by light;
FIG. 2 is a comparison view of an influence on the inhibition of melatonin secretion per unit brightness;
FIG. 3 is a schematic plan view of a lighting apparatus according to Embodiment 1 of the present invention;
FIG. 4 is a schematic cross-sectional view taken along the line IV-IV of FIG. 3;
FIG. 5 is a view of a vertical light distribution curve of a first luminous portion;
FIG. 6 is a view of the vertical light distribution curve of a second luminous portion;
FIG. 7 is a block diagram showing a configuration of a control system of the lighting apparatus according to Embodiment 1;
FIG. 8 is an example of a flow chart showing a process procedure for turning-on control in the lighting apparatus;
FIG. 9A is a timing chart showing a control process of turning-on and a luminous intensity of the first luminous portion;
FIG. 9B is a timing chart showing a control process of the turning-on and the luminous intensity of the second luminous portion;
FIG. 10 is another example of the flow chart showing the process procedure for the turning-on control in the lighting apparatus;
FIG. 11A is a timing chart showing the control process of the turning-on and the luminous intensity of the first luminous portion;
FIG. 11B is a timing chart showing the control process of the turning-on and the luminous intensity of the second luminous portion;
FIG. 12 is still another example of the flow chart showing the process procedure for the turning-on control in the lighting apparatus;
FIG. 13A is a timing chart showing the control process of the turning-on and the luminous intensity of the first luminous portion;
FIG. 13B is a timing chart showing the control process of the turning-on and the luminous intensity of the second luminous portion;
FIG. 14 is a schematic view showing another configuration of a lighting apparatus;
FIG. 15 is a schematic view showing another configuration of the lighting apparatus;
FIG. 16 is a schematic view showing still another configuration of a lighting apparatus;
FIG. 17 is a schematic view showing still another configuration of the lighting apparatus;
FIG. 18 is a view of a vertical light distribution curve of a second luminous portion;
FIG. 19 is an explanatory view for explaining the amount of light entering eyes of a user at one scene in daily life;
FIG. 20 is an explanatory view for explaining the amount of light entering the eyes of the user at another scene in daily life;
FIG. 21 is an explanatory view for explaining the amount of light entering the eyes of the user at still another scene in daily life;
FIG. 22 shows an example in which a lighting mode of the lighting apparatus is set to a study mode;
FIG. 23 shows an example in which the lighting mode of the lighting apparatus is set to a dining mode;
FIG. 24 shows an example in which the lighting mode of the lighting apparatus is set to a television-viewing mode;
FIG. 25 shows an example in which the lighting mode of the lighting apparatus is set to a relaxation mode;
FIG. 26 is a schematic view showing yet another configuration of a lighting apparatus;
FIG. 27 is a schematic view showing still another configuration of a lighting apparatus;
FIG. 28A is an explanatory view for explaining an operation of the lighting apparatus; and
FIG. 28B is an explanatory view for explaining the operation of the lighting apparatus.

### [Explanation of the Reference Numerals]

3 first luminous portion (luminous portion)
32 shield
4 second luminous portion (luminous portion)
61 control portion (control means)
61 clock portion
8 illuminance sensor

### [Best Mode for Carrying Out the Invention]

The present invention will be described hereinbelow in detail on the basis of the drawings illustrating its embodiments.

### (Embodiment 1)

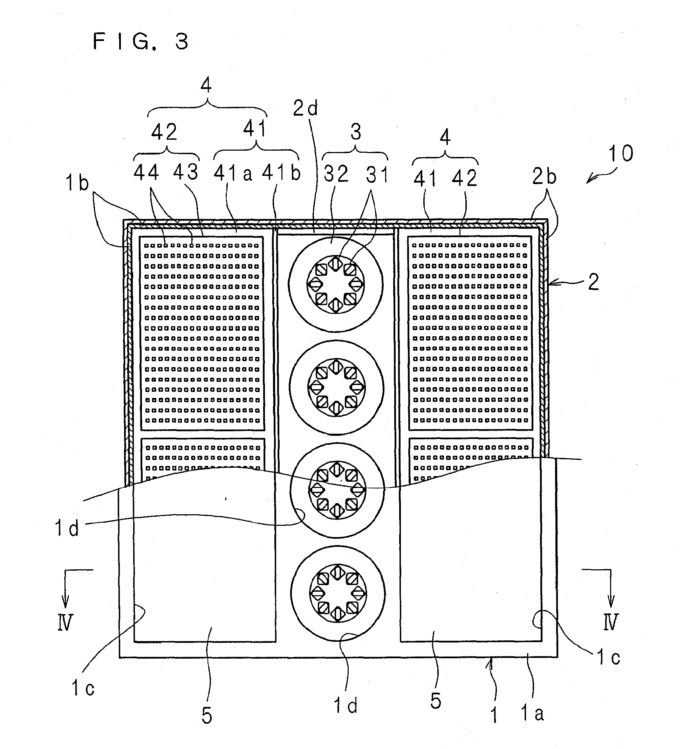

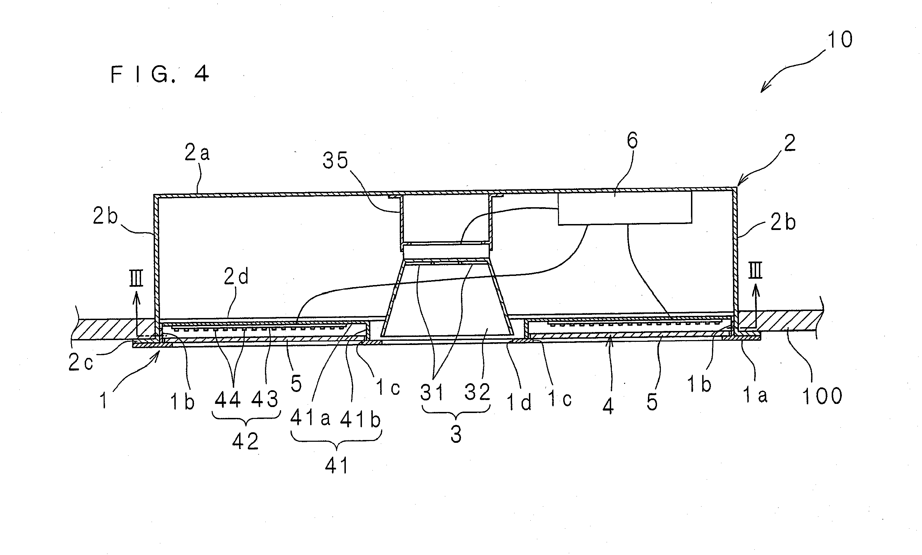

FIG. 3 is a schematic plan view of a lighting apparatus 10 according to Embodiment 1 of the present invention, and FIG. 4 is a schematic cross-sectional view taken along the line IV-IV of FIG. 3. It is to be noted that a part of FIG. 3 is illustrated by using a partial cross-section taken along the line III-III of FIG. 4.

In the drawings, the reference numeral 1 denotes a cover made of a resin or a metal, and the cover 1 includes a rectangular plate portion 1a and four side walls 1b, lb ··· which extend from a peripheral edge part of the plate portion 1a in one direction substantially orthogonal to the plate portion 1a. The plate portion 1a is formed with two rectangular windows 1c and 1c along two sides thereof which oppose each other. In addition, the plate portion 1a is formed with a plurality of (four in the drawing) circular windows 1d, Id ··· at the same intervals between the rectangular windows 1c and 1c along the rectangular windows 1c and 1c. Onto the cover 1, a metal frame 2 is fitted externally, and the cover 1 and the frame 2 constitute a case of the lighting apparatus 10.

The frame 2 includes a rectangular bottom plate portion 2a, four rectangular side walls 2b, 2b ··· which extend from end edges of the bottom plate portion 2a in one direction substantially orthogonal to the bottom plate portion 2a, and a peripheral edge portion 2c which is parallel with the bottom plate portion 2a, and extends outwardly from end edges of the four side walls 2b, 2b ···. On an opening side of the two side walls 2b and 2b which oppose each other, slender rectangular plate-like attachment portions 2d and 2d in parallel with the bottom plate portion 2a are formed over substantially entire lengths of the side walls 2b and 2b.

To the frame 2, a plurality of (four in the drawing) first luminous portions 3, 3 ··· which emit light with high directivity are attached via a support body 35 such that the first luminous portions 3, 3 ··· re parallel with the two opposing side walls 2b and 2b of the frame 2, and are at the same positions as those of the circular windows 1d, 1d ··· provided in the cover 1 when the frame 2 is fitted onto the cover 1. The first luminous portion 3 includes a plurality of (eight in the drawing) LED modules 31, 31 ··· which are provided at the same intervals in a circumferential direction, and a shield 32 which shields part of light emitted from the plurality of LED modules 31, 31 ···.

The LED module 31 includes a rectangular ceramic board, a plurality of (e.g., thirty-six) LED elements which are thickly mounted in the central part on one surface of the ceramic board, a sealing resin for sealing the plurality of LED elements in which a fluorescent material is dispersed, and input and output terminals, and is configured such that its luminous color is a light bulb color. The shield 32 has a cylindrical shape in which an inner diameter is successively reduced, and the side of the shield 32 where the inner diameter is reduced corresponds to the side with the LED modules 31,31 ···, and the shield 32 is provided so as to surround the LED modules 31, 31 ···.

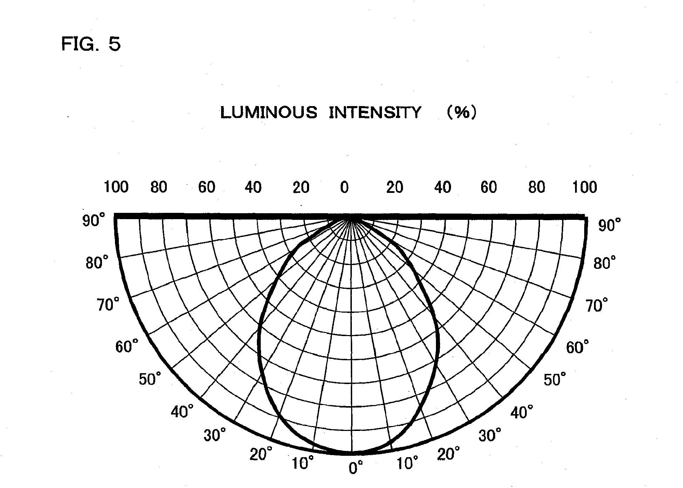

FIG. 5 is a view of a vertical light distribution curve showing a distribution of luminous intensities (light distribution) with respect to individual directions of the first luminous portion 3. In the drawing, the circumferential direction indicates an angle formed with a vertical line (optical axis) passing through a light center of the first luminous portion 3, while the radial direction indicates a luminous intensity relatively presented on the basis of the assumption that the maximum value is 100%. As shown in the drawing, all light emitted from the thus-configured first luminous portions 3, 3 ··· is included in the range where the angle formed with the optical axis is not more than 60°.

Further, in the frame 2, a plurality of (two in the drawings) second luminous portions 4 and 4 which emit light with low directivity are fixed by being attached to the attachment portions 2d and 2d along the two opposing side walls 2b and 2b so as to be at the same positions as those of the rectangular windows 1c and 1c provided in the cover 1 when the frame 2 is fitted onto the cover 1. The second luminous portion 4 includes a support plate 41 having a rectangular flat plate portion 41a and a rectangular side wall 41b extending from one end edge on the side with a long side of the flat plate portion 41a in a direction orthogonal to the flat plate portion 41a, and a plurality of (two in the drawing) LED modules 42 and 42 which are attached to the support plate 41.

The LED module 42 has an LED board 43 in a rectangular flat plate-like shape made of an epoxy resin, and a plurality of LEDs 44, 44 ··· mounted on the LED board 43, and is fixed to be in contact with a substantially entire surface of the flat plate portion 41a such that the longitudinal direction of the LED module 42 is in parallel with the side wall 41b of the support plate 41. As the LEDs 44, 44
···, LEDs which emit light having a daylight white color (color temperature of 5000 K) and a high color rendering property are used. The color rendering property denotes a property of a light source which influences the way a color looks, and a color of an object looks more natural as the color rendering property is better (higher color rendering property).

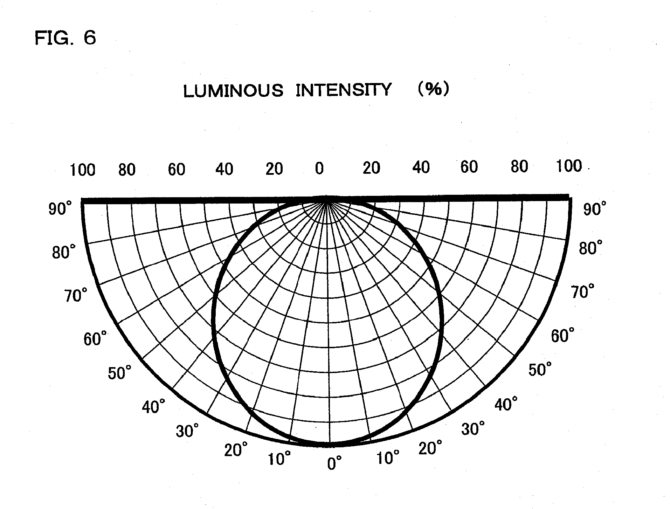

FIG. 6 is a view of the vertical light distribution curve of the LED module 42 of the second luminous portion 4. In the drawing, the circumferential direction indicates an angle formed with the optical axis of the LED module 42, while the radial direction indicates the luminous intensity relatively presented on the basis of the assumption that the maximum value is 100%. Light emitted from the thus-configured LED modules 42, 42 ··· represents a Lambert distribution which is light distribution characteristics of a typical LED, as shown in the drawing.

To the rectangular windows 1c and 1c provided in the cover 1, there are attached rectangular diffusion plates 5 and 5 which are slightly larger than the rectangular windows 1c and 1c. The diffusion plates 5 and 5 are made of a milk-white resin to which a dispersing agent is added, and an example of the milk-white resin includes a polycarbonate resin.

The thus-configured lighting apparatus 10 is attached to a ceiling 100 with its side with the diffusion plates 5 and 5 being faced downward. It is to be noted that, inside the lighting apparatus 10, there is provided a power source control circuit portion 6 including various circuit components such as a power transformer, a resistor, a capacitor, and the like, and an external power source is connected to the power source control circuit portion 6. The power source control circuit portion 6 is also connected to the first luminous portions 3, 3 ··· and the second luminous portions 4 and 4 by individual leads.

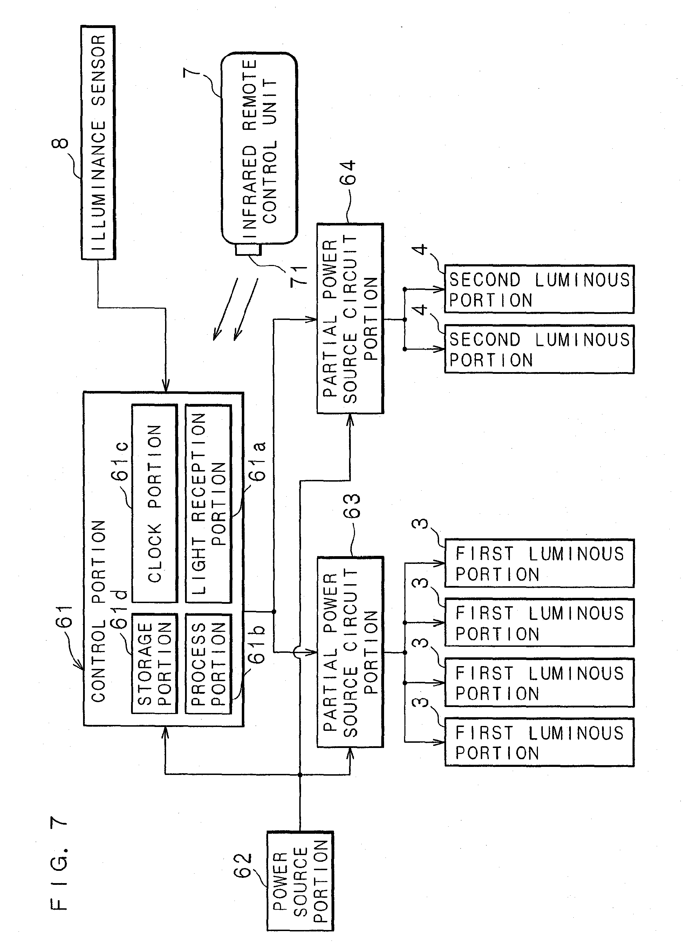

The turning-on of the first luminous portions 3, 3 ··· and the second luminous portions 4 and 4 is controlled by a control portion 61 provided in the power source control circuit portion 6. FIG. 7 is a block diagram showing a configuration of a control system of the lighting apparatus 10 according to Embodiment 1.

To the control portion 61, a power source portion 62 provided also in the power source control circuit portion 6 is connected. The power source portion 62 rectifies an alternating current supplied from the external power source to a direct current, and supplies the direct current to the control portion 61 and partial power source circuit portions 63 and 64.

The control portion 61 includes a light reception portion 61a which receives an infrared signal from an infrared remote control unit 7, a process portion 61b which performs a process for controlling the turning-on of the first luminous portions 3, 3 ···, and the second luminous portions 4 and 4, a clock portion 61c which measures time, and a storage portion 61d which stores a control program.

The infrared remote control unit 7 has a case in a substantially rectangular plate-like shape of a size which allows a user to carry. On one surface of the case, there is provided a power switch which accepts an operation of turning on/off the lighting apparatus and, on one end of the case, there is provided a transmission portion 71 which performs transmission of the infrared signal as a turning-on signal/a turning-off signal in accordance with an operation of the power switch. The light reception portion 61a is an infrared sensor including a light reception element for detecting an infrared ray, and receives the infrared signal transmitted from the transmission portion 71 of the infrared remote control unit 7.

When receiving the infrared signal as the turning-on signal/the turning-off signal from the infrared remote control unit 7 in the light reception portion 61a, the control portion 61 is configured to read time from the clock portion 61c and the control program stored in the storage portion 61d from the storage portion 61d into the process portion 61b, and cause the process portion 61b to issue a control instruction according to the control program to turn on/off the first luminous portions 3, 3 ··· and the second luminous portions 4 and 4.

In addition, the control portion 61 is supplied with an illuminance detected by an illuminance sensor 8 provided inside the lighting apparatus 10. The control portion 61 is configured to turning on/off the first luminous portion 3, 3 ··· and the second luminous portions 4 and 4 according to the given illuminance. It is to be noted that the control portion 61 may also be configured to regulate respective luminous intensities of the first luminous portions 3, 3 ··· and the second luminous portions 4 and 4 according to the given illuminance.

Further, to the control portion 61, the partial power source circuit portions 63 and 64 are connected. The control portion 61 generates, in the process portion 61b, a pulse signal determined in order to switch between turning-on and turning-off of the first luminous portions 3, 3 ··· and the second luminous portions 4 and 4, and in order to regulate the luminous intensities thereof when the first luminous portions 3, 3 ··· and the second luminous portions 4 and 4 are turned on, and supplies the generated pulse signal to each of the partial power source circuit portions 63 and 64. Each of the partial power source circuit portions 63 and 64 includes a switching element, and operates to open and close the switching element according to the pulse signal supplied from the control portion 61. As a result, a pulse-like current in accordance with the opening and closing operations of the switching element is supplied to each of the first luminous portions 3, 3 ··· and the second luminous portions 4 and 4 so that the first luminous portions 3, 3 ··· and the second luminous portions 4 and 4 are turned on in accordance with the supply of the current.

In the present embodiment, a configuration is adopted in which the operations of the partial power source circuit portions 63 and 64, and the turning-on of the plurality of luminous portions (the first luminous portions 3, 3 ··· and the second luminous portions 4 and 4) are controlled by one control portion 61. However, a configuration may also be adopted in which one control portion is provided for each of the plurality of luminous portions, and each control portion controls each of the plurality of luminous portions.

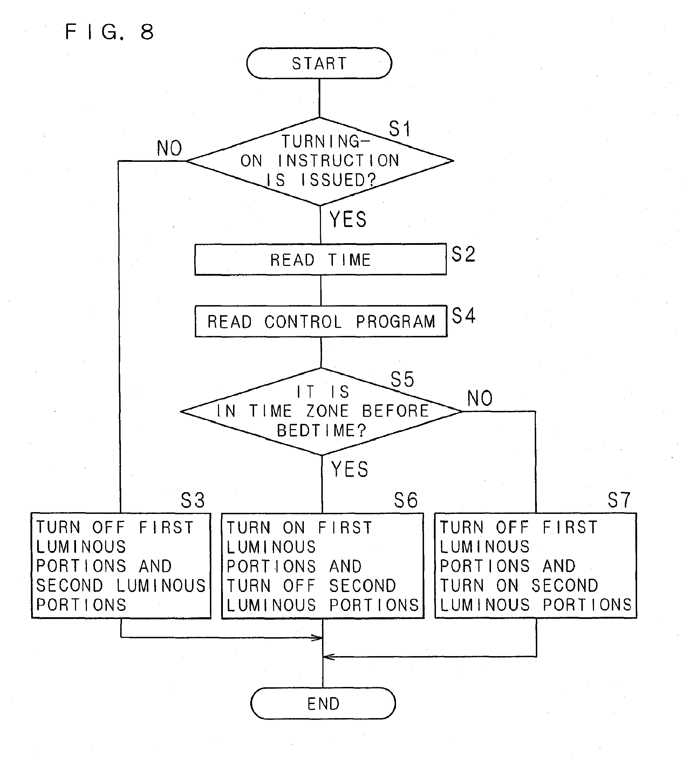

FIG. 8 is an example of a flow chart showing a process procedure for turning-on control in the lighting apparatus 10. After being turned on, the control portion 61 determines whether or not a turning-on instruction is issued using the process portion 61b (step S1). It is determined whether or not the turning-on instruction is issued by whether the latest infrared signal received by the light reception portion 61a from the infrared remote control unit 7 is the turning-on signal or the turning-off signal while the control portion 61 is kept turned on.

In the step S1, when it is determined that the turning-on instruction is issued (step S1: YES), that is, when the latest infrared signal received by the light reception portion 61a from the infrared remote control unit 7 is the turning-on signal, the control portion 61 reads time from the clock portion 61c into the process portion 61b (step S2).

On the other hand, in the step S1, when it is determined that the turning-on instruction is not issued (step S1: NO), that is, when the latest infrared signal received by the light reception portion 61a from the infrared remote control unit 7 is the turning-off signal, the operation of the turning-on control in the lighting apparatus 10 is ended in a state where the first luminous portions 3, 3 ··· and the second luminous portions 4 and 4 are kept turned off (step S3).

Then, the control portion 61 reads the control program stored in the storage portion 61d into the process portion 61b (step S4).

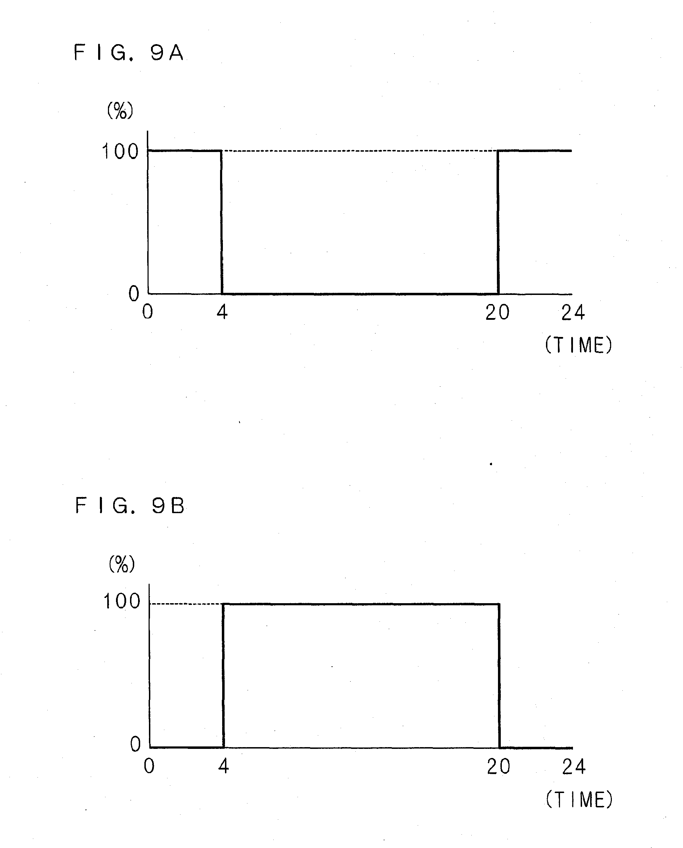

FIGS. 9A and 9B are timing charts showing control processes for the turning-on and respective luminous intensities of the first luminous portions 3, 3 ··· and the second luminous portions 4 and 4 which are set by the control program. FIG. 9A shows the timing chart for the first luminous portions 3,3 ···, while FIG. 9B shows the timing chart for the second luminous portions 4 and 4.

In each of FIGS. 9A and 9B, the horizontal axis indicates time, while the vertical axis indicates respective luminous intensities of the first luminous portions 3, 3 ··· and the second luminous portions 4 and 4 which are relatively presented on the basis of the assumption that the maximum value is 100%. In a time zone from 8 PM to 4 AM which is set as a time zone before bedtime, the first luminous portions 3, 3 ··· are turned on, while the second luminous portions 4 and 4 are turned off. On the other hand, in a time zone from 4 AM to 8 PM which is set as a normal time zone, the first luminous portions 3, 3 ··· are turned off, while the second luminous portions 4 and 4 are turned on.

Next, the control portion 61 determines whether or not it is in the time zone before bedtime by using the time read in the step S2 and the control program read in the step 4 (step S5).

In the step S5, when it is determined that it is in the time zone before bedtime (step S5: YES), the first luminous portions 3, 3 ··· are turned on, the second luminous portions 4 and 4 are turned off (step S6), and the operation of the turning-on control in the lighting apparatus 10 is ended.

On the other hand, in the step S5, when it is determined that it is not in the time zone before bedtime (step S5: NO), the first luminous portions 3, 3 ··· are turned off, the second luminous portions 4 and 4 are turned on (step S7), and the operation of the turning-on control in the lighting apparatus 10 is ended.

In the thus-configured lighting apparatus 10 according to the present embodiment, light emitted from the first luminous portions 3, 3 ··· becomes light with high directivity all of which is included in the range where the angle formed with the optical axis is not more than 60° by the shield 32 provided on the side with outgoing surfaces of the LED modules 31, 31 ···. On the other hand, light emitted from the second luminous portions 4 and 4 is diffused by the diffusion plates 5 and 5 to become uniform light with which the entire room is illuminated. The light emitted from the first luminous portions 3, 3 ··· is limited in its direction, but the light spreads to a certain extent so that it is possible to obtain a sufficient illuminance on an illuminated surface illuminated with the light from the first luminous portions 3, 3 ···, e.g., on a floor surface. In the time zone before bedtime, since the first luminous portions 3, 3 ··· are turned on, and the second luminous portions 4 and 4 are turned off, the room is illuminated with sufficient brightness, and the amount of light emitted in a direction (substantially horizontal direction) toward eyes of a user in a normal usage mode is suppressed to be small. On the other hand, in the normal time zone, since the first luminous portions 3, 3 ··· are turned off, and the second luminous portions 4 and 4 are turned on, the room is illuminated with sufficient brightness, and the lighting includes the light emitted in the direction (substantially horizontal direction) toward the eyes of the user in the normal usage mode.

As a result, in the time zone before bedtime, it is possible to suppress the amount of light in a wavelength range inhibiting melatonin secretion to be small, and scarcely inhibit melatonin secretion to encourage smooth initiation of sleep, while in the morning, it is possible to inhibit melatonin secretion to encourage smooth awakening. Accordingly, it is possible to perform adequate lighting in accordance with time. In addition, the first luminous portions 3, 3 ··· are configured to emit light having the light bulb color, and the second luminous portions 4 and 4 are configured to emit light having the daylight while color. Consequently, in the normal time zone, lighting is performed by using light having a low color temperature, i.e., light having the light bulb color which scarcely includes the wavelength range in the vicinity of 464 nm which is influential in inhibiting melatonin secretion, while in the time zone before bedtime, lighting is performed by using light having a high color temperature, i.e., light having the daylight white color which abundantly includes the wavelength range in the vicinity of 464 nm. Accordingly, it is possible to change the amount of light in the wavelength range inhibiting melatonin secretion which enters the eyes of the user, and further change an influence on the inhibition of melatonin secretion.

In Embodiment 1 described above, the first luminous portions 3, 3 ··· are luminous portions (light sources) each having high directivity, and the second luminous portions 4 and 4 are luminous portions (light sources) each having low directivity. However, the plurality of luminous portions may be any plurality of luminous portions which have different directivities. In a case where light sources each having relatively high directivity are set as the first luminous portions, and light sources each having relatively low directivity are set as the second luminous portions, even when the degree of a difference between the directivities is different, it is possible to achieve the effect described above. The same applies to the description of each of the following embodiments.

In addition, the view of the vertical light distribution curve of each of the first luminous portions 3, 3 ··· and the second luminous portions 4 and 4 shown in FIGS. 5 and 6 are only illustrative, and light distribution characteristics are not limited thereto. That is, the light distribution characteristics may be any light distribution characteristics in which the illuminance is reduced more sharply from the position immediately below the luminous portion (the angle formed with the optical axis is 0°) toward the horizontal direction (the angle formed with the optical axis is 90°) in the light distribution characteristics of the first luminous portion than in those of the second luminous portion.

Further, since a configuration is adopted in which the respective luminous intensities of the first luminous portions 3, 3 ··· and the second luminous portions 4 and 4 are controlled according to time, it is possible to automatically change the influence on the inhibition of melatonin secretion without setting by the user, and perform adequate lighting in accordance with time. Furthermore, since simple control is adopted in which either one of each of the first luminous portions 3, 3 ··· and each of the second luminous portions 4 and 4 is turned on, it is possible to simplify a circuit configuration.

### (Embodiment 2)

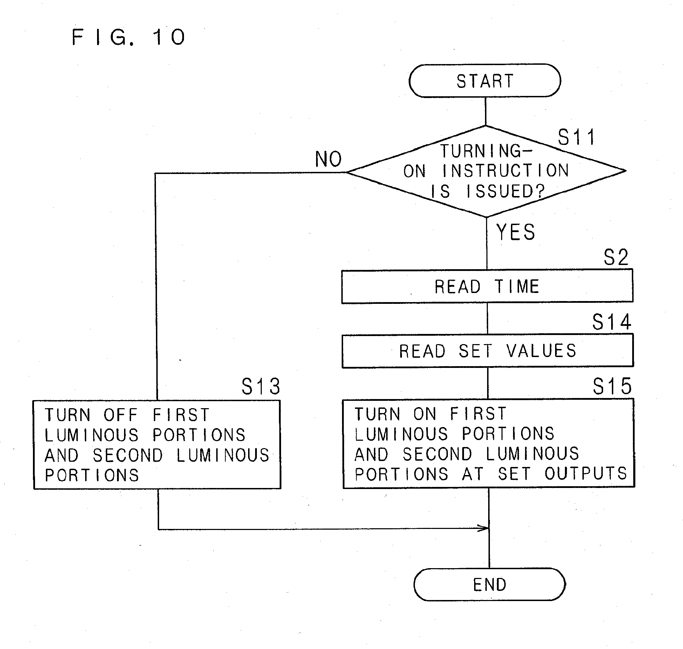

FIG. 10 is another example of the flow chart showing the process procedure for the turning-on control in the lighting apparatus 10. Since the other configuration is the same as that described in Embodiment 1, the drawing and the description thereof will be omitted.

After being turned on, the control portion 61 determines whether or not the turning-on instruction is issued using the process portion 61b (step S11).

In the step S11, when it is determined that the turning-on instruction is issued (step S11: YES), the control portion 61 reads time from the clock portion 61c into the process portion 61b (step S12).

On the other hand, in the step S11, when it is determined that the turning-on instruction is not issued (step S11: NO), the operation of the turning-on control in the lighting apparatus 10 is ended in a state where the first luminous portions 3, 3 ··· and the second luminous portions 4 and 4 are kept turned off (step S13).

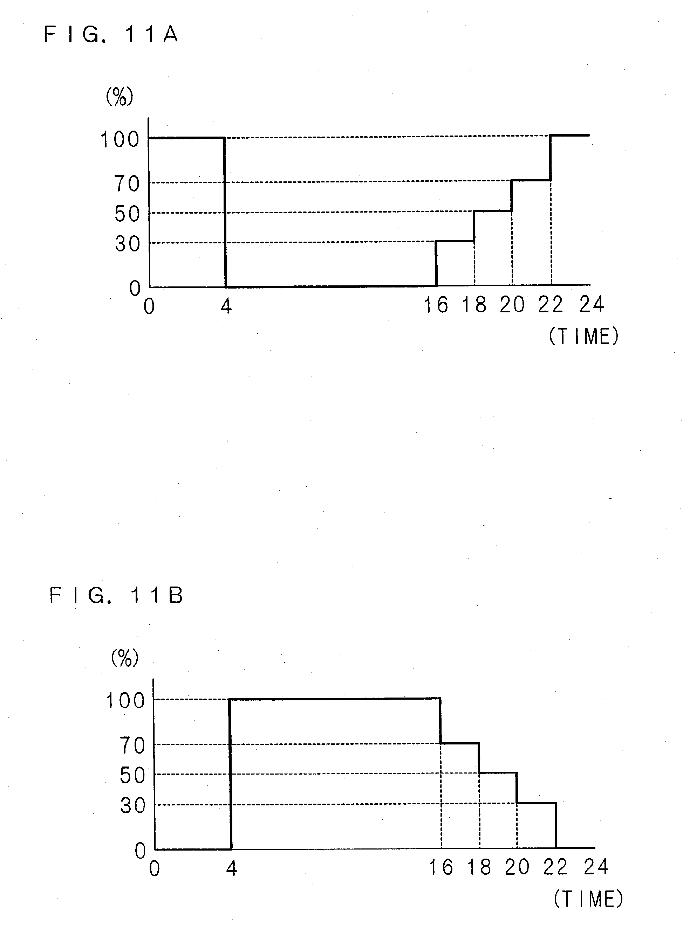

Then, the control portion 61reads set values for the first luminous portions 3, 3 ··· and the second luminous portions 4 and 4 which are in correspondence to the time read in the step S12 from the storage portion 61d into the process portion 61b (step S14). FIGS. 11A and 11B are timing charts showing control processes for the turning-on and respective luminous intensities of the first luminous portions 3, 3 ··· and the second luminous portions 4 and 4 which are stored in the storage portion 61d. FIG. 11A shows the timing chart for the first luminous portions 3, 3 ···, while FIG. 11B shows the timing chart for the second luminous portions 4 and 4.
In each of FIGS. 11A and 11B, the horizontal axis indicates time, while the vertical axis indicates respective luminous intensities of the first luminous portions 3, 3 ··· and the second luminous portions 4 and 4 which are relatively presented on the basis of the assumption that the maximum value is 100%. It is to be noted that the first luminous portions 3, 3 ··· and the second luminous portions 4 and 4 are configured to have a constant illuminance on an illuminated surface illuminated with light from the first luminous portions 3, 3 ··· and the second luminous portions 4 and 4, e.g., on a floor surface immediately below the lighting apparatus irrespective of time.

Next, the control portion 61 turns on the first luminous portions 3, 3 ··· and the second luminous portions 4 and 4 using set outputs so as to achieve the set values read in the step S14 (step S15), and ends the operation of the turning-on control in the lighting apparatus 10.

As described above, by controlling the turning-on of the first luminous portions 3, 3 ··· and the second luminous portions 4 and 4, it is possible to scarcely inhibit melatonin secretion to encourage smooth initiation of sleep in the time zone before bedtime, while in the morning, it is possible to inhibit melatonin secretion to encourage smooth awakening. Accordingly, it is possible to perform adequate lighting in accordance with time. In addition, in the time zone from 4 PM to 10PM, the respective luminous intensities of the first luminous portions 3, 3 ··· and the second luminous portions 4 and 4 are changed stepwise so that the user dose not feel discomfort. In addition, irrespective of time, the lighting apparatus is configured to have the constant illuminance on the illuminated surface illuminated with light from the first luminous portions 3, 3 ··· and the second luminous portions4 and 4, e.g., on the floor surface immediately below the lighting apparatus so that sufficient brightness can be obtained constantly.

### (Embodiment 3)

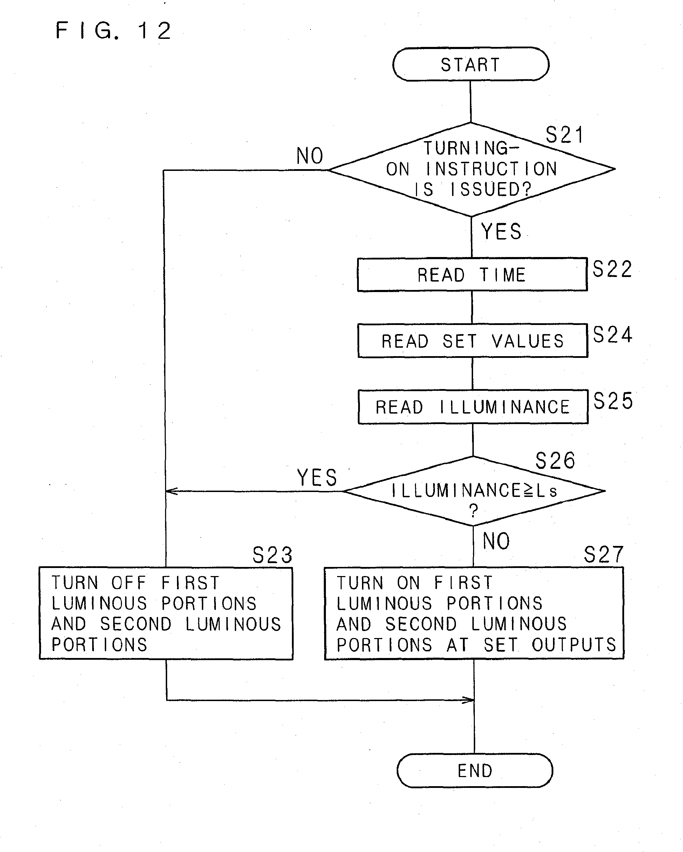

FIG. 12 is still another example of the flow chart showing the process procedure for the turning-on control in the lighting apparatus 10. Since the other configuration is the same as that described in Embodiment 1, the drawing and description thereof will be omitted.

After being turned on, the control portion 61 determines whether or not the turning-on instruction is issued using the process portion 61b (step S21).

In the step S21, when it is determined that the turning-on instruction is issued (step S21: YES), the control portion 61 reads time from the clock portion 61c into the process portion 61b (step S22).

On the other hand, in the step S21, when it is determined that the turning-on instruction is not issued (step S21: NO), the operation of the turning-on control in the lighting apparatus 10 is ended in a state where the first luminous portions 3, 3 ··· and the second luminous portions 4 and 4 are kept turned off (step S23).

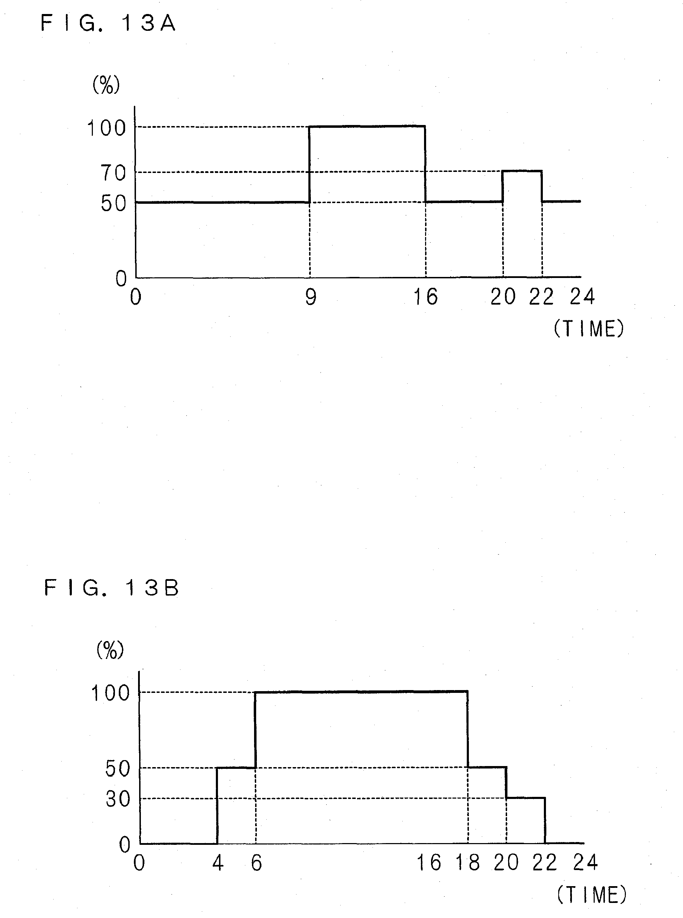

Then, the control portion 61 reads set values for the first luminous portions 3, 3 ··· and the second luminous portions 4 and 4 which are in correspondence to the time read in the step S22 from the storage portion 61d into the process portion 61b (step S24). FIGS. 13A and 13B are timing charts showing control processes for the turning-on and the respective luminous intensities of the first luminous portions 3, 3 ··· and the second luminous portions 4 and 4 which are stored in the storage portion 61d. FIG. 13A shows the timing chart for the first luminous portions 3, 3 ···, while FIG. 13B shows the timing chart for the second luminous portions 4 and 4.
In each of FIGS. 13A and 13B, the horizontal axis indicates time, while the vertical axis indicates respective luminous intensities of the first luminous portions 3, 3 ··· and the second luminous portions 4 and 4 which are relatively presented on the basis of the assumption that the maximum value is 100%. It is to be noted that the first luminous portions 3, 3 ··· and the second luminous portions 4 and 4 are configured such that the illuminance on the illuminated surface illuminated with light from the first luminous portions 3, 3 ··· and the second luminous portions 4 and 4, e.g., on the floor surface immediately below the lighting apparatus changes in accordance with time.

Next, the control portion 61 reads the illuminance detected by the illuminance sensor 8 (step S25), and determines whether or not the read illuminance is not less than a predetermined value Ls (step S26). It is to be noted that the predetermined value Ls is set on the basis of an illuminance when the first luminous portions 3, 3 ··· and the second luminous portions 4 and 4 are turned on according to the set values read in the step S24.

In the step S26, when it is determined that the illuminance is not less than the predetermined value Ls (step S26: YES), the operation of the turning-on control in the lighting apparatus 10 is ended in a state where the first luminous portions 3, 3 ··· and the second luminous portions 4 and 4 are kept turned off (step S23).

On the other hand, in the step S26, when it is determined that the illuminance is less than the predetermined value Ls (step S26: NO), the first luminous portions 3, 3 ··· and the second luminous portions 4 and 4 are turned on using set outputs so as to achieve the set values read in the step S24 (step S27), and the operation of the turning-on control in the lighting apparatus 10 is ended.

As described above, by controlling the turning-on of the first luminous portions 3, 3 ··· and the second luminous portions 4 and 4, in the time zone before bedtime, it is possible to scarcely inhibit melatonin secretion to encourage smooth initiation of sleep, while in the morning, it is possible to inhibit melanin secretion to encourage smooth awakening. In addition, since the respective luminous intensities of the first luminous portions 3, 3 ··· and the second luminous portions 4 and 4 are changed in accordance with time, it is possible to obtain brightness sufficient enough to conduct delicate work in the daytime, while in the time zone from 10 PM to 4 AM, it is possible to suppress the luminous intensities to be low to bring melatonin secretion into a further natural state to perform adequate lighting in accordance with time.

Further, since the respective luminous intensities of the first luminous portions 3, 3 ··· and the second luminous portions 4 and 4 are controlled in accordance with the illuminance detected by the illuminance sensor 8, and the first luminous portions 3, 3 ··· and the second luminous portions 4 and 4 are turned on/off in accordance with the illuminance, it is possible to perform adequate lighting, and also contribute to energy conservation. The control portion 61 is configured to turn on/off the first luminous portions 3, 3 ··· and the second luminous portions 4 and 4 in accordance with the illuminance. However, the control portion 61 may also be configured to regulate the respective luminous intensities of the first luminous portions 3, 3 ··· and the second luminous portions 4 and 4 in accordance with the illuminance.

In the lighting apparatus according to each of Embodiments 1 to 3, the first luminous portions 3, 3 ··· and the second luminous portions 4 and 4 are configured to emit light with the light bulb color and light with the daylight white color, respectively. However, the luminous color is not limited thereto, and the luminous portions can be configured to have appropriate luminous colors in accordance with an environment where the lighting apparatus is used, and the use of the lighting apparatus. For example, the first luminous portions 3, 3 ··· may be configured to have the daylight white color or the daylight color (e.g., 6800 K) as the luminous color, and the second luminous portions 4 and 4 may also be configured to have the light bulb color or the daylight color as the luminous color.

In addition, a configuration may also be adopted in which the first luminous portions 3, 3 ··· and/or the second luminous portions 4 and 4 include three types of LEDs with different luminous colors which are blue, green, and the light bulb color LEDs, and the luminous intensity of each of the three types of LEDs can be changed. With the configuration, it is possible to freely change a color temperature on an illuminated surface illuminated with light from the first luminous portions 3, 3 ··· and/or the second luminous portions 4 and 4 over a wide range (e.g., from the light bulb color to the daylight white color). It is to be noted that the number of types of the LEDs with different luminous colors is not limited to three, and the combination of other colors may also be adopted.

Further, as the blue LED, there may also be used two blue LEDs having different peak wavelengths which are a first blue LED for emitting light with the peak wavelength in the wavelength range in the vicinity of 464 nm which is influential in inhibiting melatonin secretion, and a second blue LED for emitting light without the peak wavelength in the wavelength range in the vicinity of 464 nm. In the time zone before bedtime, the second blue LED is turned on, while in the time zone other than the time zone before bedtime, the first blue LED is turned on, whereby it is possible to change the amount of light in the wavelength range inhibiting melatonin secretion which enters the eyes of a user to further change the influence on the inhibition of melatonin secretion.

Furthermore, in particular, when the second blue LED is used in the first luminous portion, and the first blue LED is used in the second luminous portion, it follows that a light source having a slight influence on the inhibition of melatonin secretion is used in the first luminous portion of which the luminous intensity is increased in the time zone before bedtime. Therefore, a user is able to get to sleep smoothly. As the peak wavelength of the first blue LED, 464 nm, which is the most influential in inhibiting melatonin, is optimum. However, the peak wavelength is not limited thereto, and the peak wavelength may be any wavelength in the vicinity of 464 nm.

Moreover, in the lighting apparatus according to each of Embodiments 1 to 3, the control portion 61 and the infrared remote control unit 7 are configured to accept only the turning on/off operation of the power switch by a user. However, the operation to be accepted thereby is not limited thereto, and a configuration may also be adopted in which a user can freely set the timing charts showing the control processes for the turning-on and the respective luminous intensities of the first luminous portions 3, 3 ··· and the second luminous portions 4 and 4 in accordance with the lifestyle of the user.

### (Embodiment 4)

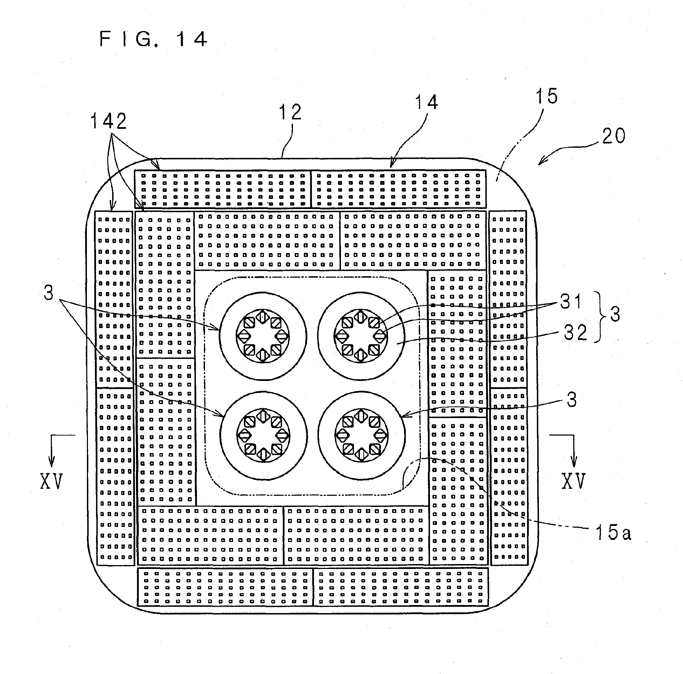

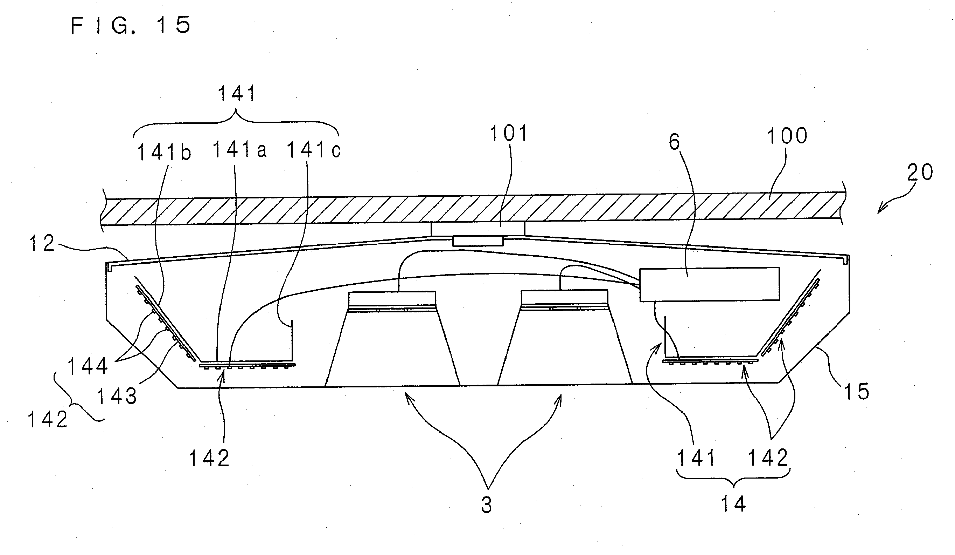

FIGS. 14 and 15 are schematic views each showing another configuration of a lighting apparatus. FIG. 14 shows the disposition of the luminous portions viewed from the outgoing side of light in a lighting apparatus 20, while FIG. 15 shows the disposition of the luminous portions viewed from a cross section taken along the line XV-XV of FIG. 14. The depiction and description of the detailed configuration will be omitted for the sake of illustrative convenience.

In the drawings, the reference numeral 12 denotes a substantially rectangular plate-like metal frame, and a plurality of (four in the drawing) first luminous portions 3, 3 ··· each emitting light with high directivity are provided in the central part of the frame 12 in two rows and two columns at the same intervals. Since the first luminous portion 3 is the same as the first luminous portion 3 described in the lighting apparatus according to Embodiment 1, the description thereof will be omitted.

Additionally, in the frame 12, a second luminous portion 14 which emits light with low directivity is provided so as to surround the first luminous portions 3, 3 ···. The second luminous portion 14 includes two support plates 141 and 141 each having a rectangular flat plate portion 141a which has a rectangular window, an outer peripheral wall portion 141b which extends from an outer peripheral edge of the rectangular flat plate portion 141a to form an angle with the rectangular flat plate portion 141a, and an inner peripheral wall portion 141c which extends from an inner peripheral edge of the rectangular flat plate portion 141a in a direction substantially orthogonal to the rectangular flat plate portion 141a, and sixteen LED modules 142, 142 ··· which are attached to each of the rectangular flat plate portion 141a and the outer peripheral wall portion 141b of the support plate 141 in multiple relation (eight units each in the drawing). The LED module 142 of the second luminous portion 14 has light distribution characteristics represented in the view of the vertical light distribution curve shown in FIG. 6, similarly to the LED module 42 of the second luminous portion 4 in the lighting apparatus according to Embodiment 1.

A cover 15 made of a resin is attached to the frame 12 so as to cover the second luminous portion 14. The cover 15 is made of a milk-white resin to which a dispersing agent is added, and an example of the milk-white resin includes a polycarbonate resin. As shown in FIG. 14 using a two-dot chain line, the cover 15 has a substantially rectangular window 15a, and light is emitted from the first luminous portions 3, 3 ··· directly to the outside without being passed through the cover 15.

The thus-configured lighting apparatus 20 is attached to a ceiling 100 such that the side with the cover 15 is faced downward via a base plate 101 in a circular plate-like shape which is provided in the ceiling 100 preliminarily for attachment of an annular fluorescent lamp, and used as a substitute for the annular fluorescent lamp (e.g., Circline (registered trademark)-type fluorescent lamp). It is to be noted that, inside the lighting apparatus 20, there is provided the power source control circuit portion 6 including various circuit components such as a power transformer, a resistor, a capacitor, and the like, and an external power source is connected to the power source control circuit portion 6. In addition, the power source control circuit portion 6 is connected to the first luminous portions 3, 3 ··· and the second luminous portion 14 by individual leads.

In the thus-configured lighting apparatus 20 according to the present embodiment, light emitted from the first luminous portions 3, 3 ··· becomes light with high directivity all of which is included in a range where an angle formed with the optical axis is not more than 60° by the shield 32 provided on the side with outgoing surfaces of the LED modules 31, 31 ···, while light emitted from the second luminous portion 14 in a downward direction and a substantially horizontal direction is diffused by the cover 15 to become uniform light with which the entire room is illuminated. The light emitted from the first luminous portions 3, 3 ··· is limited in its direction, but the light spreads to a certain extent so that it is possible to obtain a sufficient illuminance on an illuminated surface illuminated with the light from the first luminous portions 3, 3 ···, e.g., on a floor surface. In addition, in the lighting apparatus 20, since the second luminous portion 14 is configured to emit light in the downward direction and the substantially horizontal direction, it is possible to illuminate the entire room including walls more uniformly and brightly.

It is to be noted that the lighting apparatus 20 is configured such that the turning-on control described in the lighting apparatus 10 according to each of Embodiments 1 to 3 can be applied thereto. In addition, the first luminous portions 3, 3 ··· and the second luminous portion 14 are also configured to have appropriate luminous colors in accordance with the environment where the lighting apparatus is used and the use of the lighting apparatus, as described above. As a result, in the time zone before bedtime, it is possible to scarcely inhibit melatonin secretion to encourage smooth initiation of sleep, while in the morning, it is possible to inhibit melatonin secretion to encourage smooth awakening. Accordingly, it is possible to perform adequate lighting in accordance with time or the lifestyle of a user.

### (Embodiment 5)

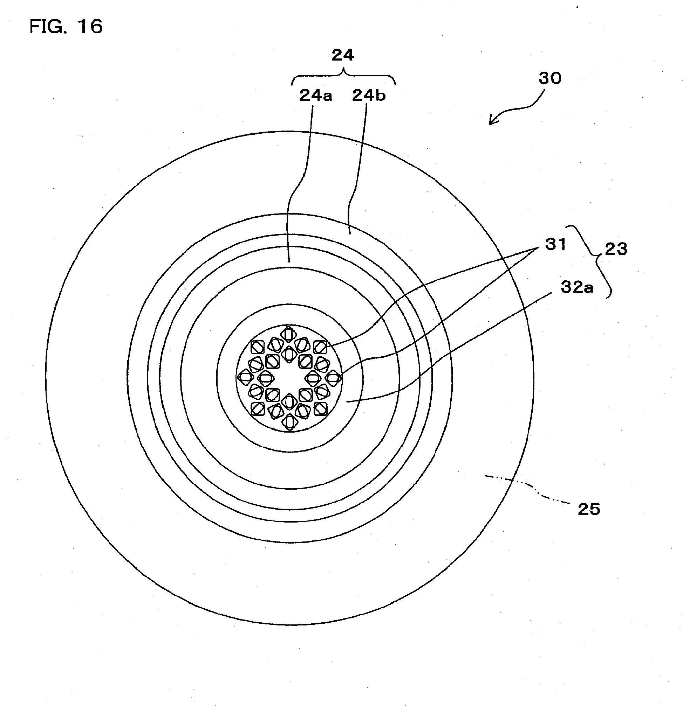

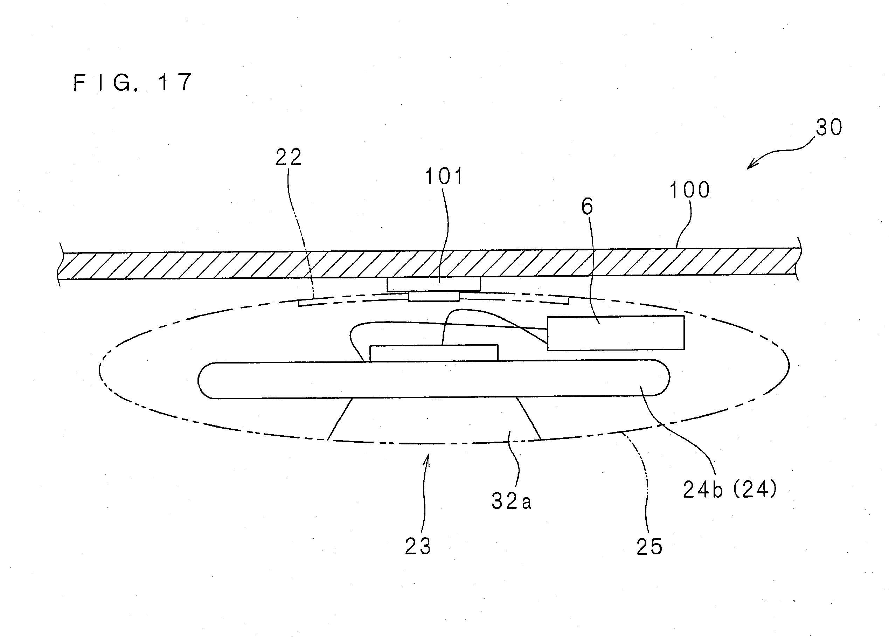

FIGS. 16 and 17 are schematic views each showing still another configuration of a lighting apparatus. FIG. 16 shows the disposition of the luminous portion viewed from the outgoing side of light of a lighting apparatus 30, while FIG. 17 shows the disposition of the luminous portion viewed from a side surface. For the sake of illustrative convenience, the depiction and description of the detailed configuration will be omitted.

In the drawing, the reference numeral 22 denotes a substantially disk-shaped metal frame, and a first luminous portion 23 which emits light with high directivity is provided in the central part of the frame 22. The first luminous portion 23 includes a plurality of (twenty-four in the drawing) LED modules 31, 31 ··· which are provided thickly, and a shield 32a which shields part of light emitted from the plurality of LED modules 31, 31 ···. Since the LED module 31 is the same as the LED module 31 described in the lighting apparatus according to Embodiment 1, the description thereof will be omitted. The shield 32a has a cylindrical shape in which an inner diameter is successively reduced, and the side of the shield 32a where the inner diameter is reduced corresponds to the side with the LED modules 31, 31 ···, and the shield 32a is provided so as to surround the LED modules 31, 31 ···.

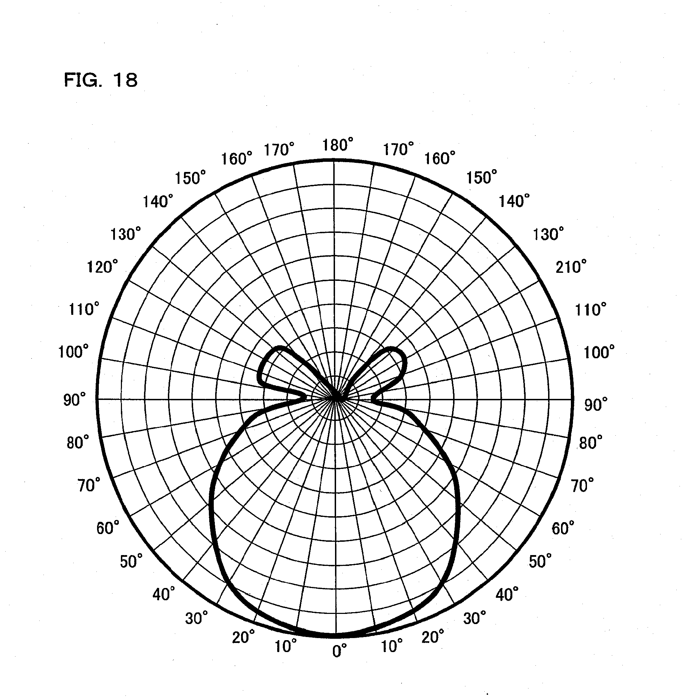

In addition, in the frame 22, a second luminous portion 24, which includes two annular fluorescent lamps 24a and 24b having different diameters and emits light with low directivity, is provided concentrically with the optical axis of the first luminous portion 23. FIG. 18 is a view of a vertical light distribution curve of the second luminous portion 24. In the drawing, the circumferential direction indicates an angle formed with the optical axis of the second luminous portion 24, and the radial direction indicates a luminous intensity relatively presented on the basis of the assumption that the maximum value is 100%. It is to be noted that, as the fluorescent lamps 24a and 24b, a fluorescent lamp of which the luminous color has the color temperature of the daylight white color or the daylight color is used.

A cover 25 made of a resin is attached to the frame 22 so as to cover the second luminous portion 24. The cover 25 is made of a milk-white resin to which a dispersing agent is added, and an example of the milk-white resin includes a polycarbonate resin. It is to be noted that the cover 25 has a circular window with a diameter substantially identical with an outer diameter of the shield 32a, and light is emitted from the first luminous portion 23 directly to the outside without being passed through the cover 25.

The thus-configured lighting apparatus 30 is attached to the ceiling 100 such that the side with the cover 25 is faced downward via the base plate 101 in a circular plate-like shape which is provided in the ceiling 100 preliminarily for attachment of an annular fluorescent lamp. It is to be noted that, inside the lighting apparatus 30, there is provided the power source control circuit portion 6 including various circuit components such as a power transformer, a resistor, a capacitor, and the like, and an external power source is connected to the power source control circuit portion 6. The power source control circuit portion 6 is also connected to the first luminous portion 23 and the second luminous portion 24 using individual leads.

In the thus-configured lighting apparatus 30 according to the present embodiment, light emitted from the first luminous portion 23 becomes light with high directivity all of which is included in a range where an angle formed with the optical axis is not more than 60° by the shield 32a provided on the side with outgoing surfaces of the LED modules 31,31 ···, while light emitted from the second luminous portion 24 in substantially all directions is diffused by the cover 25 to become uniform light with which the entire room is illuminated. The light emitted from the first luminous portion 23 is limited in its direction, but the light spreads to a certain extent so that it is possible to obtain a sufficient illuminance on an illuminated surface illuminated with the light from the first luminous portion 23, e.g., on a floor surface. In addition, in the lighting apparatus 30, since the second luminous portion 24 is configured to emit light in substantially all directions, it is possible to illuminate the entire room including walls more uniformly and brightly.

It is to be noted that the lighting apparatus 30 is configured such that the turning-on control described in the lighting apparatus 10 according to each of Embodiments 1 to 3 can be applied thereto. In addition, the first luminous portion 23 is also configured to have appropriate luminous colors in accordance with the environment where the lighting apparatus is used and the use of the lighting apparatus, as described above. As a result, in the time zone before bedtime, it is possible to scarcely inhibit melatonin secretion to encourage smooth initiation of sleep, while in the morning, it is possible to inhibit melatonin secretion to encourage smooth awakening. Accordingly, it is possible to perform adequate lighting in accordance with time or the lifestyle of a user.

In the lighting apparatus according to each of Embodiments 1 to 5 described above, the fist luminous portion is configured to have the shield in order to limit an angle range of light emitted from the first luminous portion. However, the configuration is not limited thereto, and the configuration may be any configuration capable of limiting the angle range of the light emitted from the first luminous portion to an appropriate angle range.

By turning on the first luminous portion and the second luminous portion in a switching manner using the lighting apparatus according to each of Embodiments 1 to 5, it is possible to significantly change the amount of light entering the eyes of a user. Hereinbelow, by taking the case where the lighting apparatus 20 according to Embodiment 4 is used as an example, a description will be given of a change in the amount of light entering the eyes of the user for each positional relation between the user and the lighting apparatus.

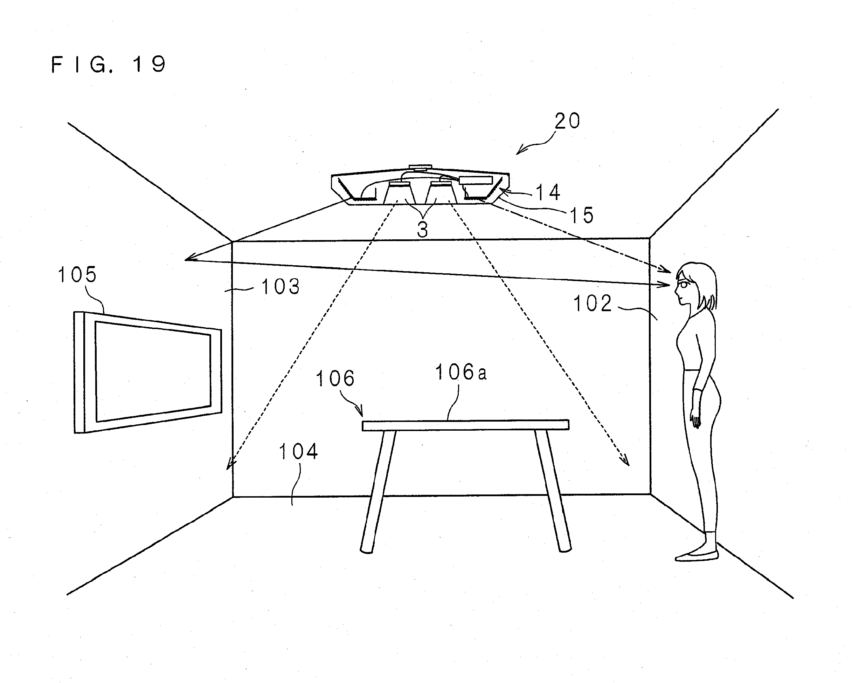

FIG. 19 is an explanatory view for explaining the amount of light entering the eyes of the user at one scene in daily life. The user stands near a wall 102 away from the lighting apparatus 20, and is watching television 105 hung on an opposing wall 103. In the following description, the first luminous portions 3, 3 ··· and the second luminous portion 14 are turned on such that illuminances thereof have substantially identical values on a floor surface 104 to compare the amounts of light entering the eyes of the user.

In this state, when only the first luminous portions 3, 3 ··· of the lighting apparatus 20 are turned on, light emitted from the first luminous portions 3, 3 ··· is included in the range where the angle formed with the optical axis is not more than 60°, as described above, so that the light does not enter the eyes of the user directly. However, the light is emitted in a direction indicated by the broken line in the drawing, reflected by an upper surface 106a of a table 106, the floor surface 104, and the like, and part of the reflected light enters the eyes of the user.

On the other hand, when only the second luminous portion 14 is turned on, light is emitted from the second luminous portion 14 in a downward direction and a substantially horizontal direction as described above, diffused by the cover 15, and emitted to the entire room so that part of the light directly enters the eyes of the user as indicated by the one-dot chain line in the drawing, the rest of the light is reflected by the wall 103 and the like as indicated by the solid line in the drawing, and part of the reflected light further enters the eyes of the user. When the illuminance in the vicinity of the eyes of the user in a case where the second luminous portion 14 is turned on is 300 lx, the illuminance in the vicinity of the eyes of the user in a case where the first luminous portions 3, 3 ··· are turned on is about 100 Ix. Consequently, it is possible to change the amount of the light entering the eyes of the user by a factor of about three only by turning on the first luminous portions 3, 3 ··· and the second luminous portion 14 in a switching manner.

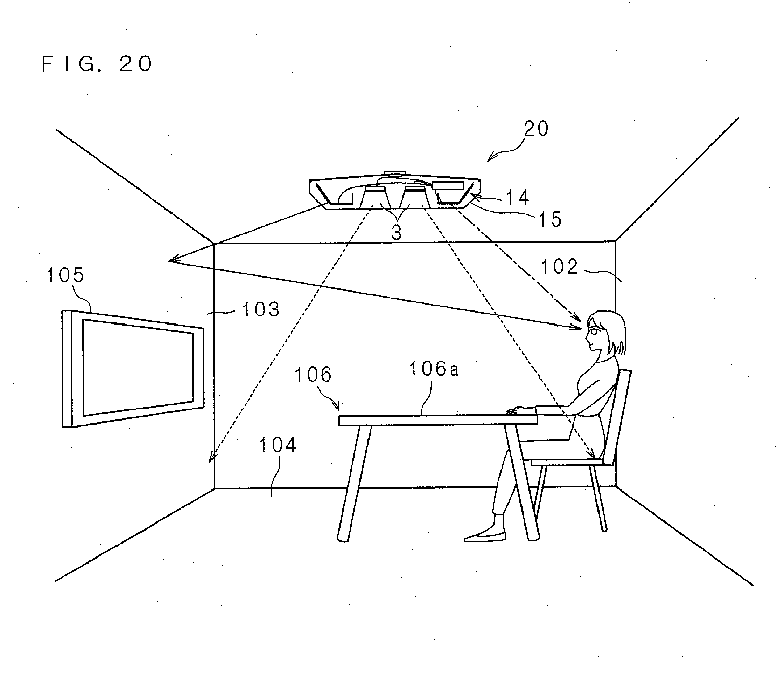

FIG. 20 is an explanatory view for explaining the amount of light entering the eyes of the user at another scene in daily life. The user sits on a chair near the wall 102 away from the lighting apparatus 20, and is watching television 105 hung on the opposing wall 103.

In this state, when only the first luminous portions 3, 3 ··· of the lighting apparatus 20 are turned on, the light emitted from the first luminous portions 3, 3 ··· scarcely enters the eyes of the user directly, but is emitted in a direction indicated by the broken line in the drawing, reflected by the upper surface 106a of the table 106, the floor surface 104, and the like, and part of the reflected light enters the eyes of the user.

On the other hand, when only the second luminous portion 14 is turned on, part of the light emitted from the second luminous portion 14 directly enters the eyes of the user as indicated by the one-dot chain line in the drawing, the rest of the light is reflected by the wall 103 and the like as indicated by the solid line in the drawing, and part of the reflected light further enters the eyes of the user. When compared with the case where the user stands in FIG. 19, the amount of the light which is emitted in a downward direction of the second luminous portion 14, and directly enters the eyes of the user increases so that the amount of the light directly entering the eyes of the user from the second luminous portion 14 increases in the case where the user sits. When the illuminance in the vicinity of the eyes of the user in a case where the second luminous portion 14 is turned on is 350 lx, the illuminance in the vicinity of the eyes of the user in a case where the first luminous portions 3, 3 ··· are turned on is about 100 Ix. Consequently, it is possible to change the amount of the light entering the eyes of the user by a factor of just over three only by turning on the first luminous portions 3, 3 ··· and the second luminous portion 14 in a switching manner.

With regard to the above-mentioned illuminance and a value of the amount of the light entering the eyes, since they change depending on conditions such as the size of the room, the position where the lighting apparatus is installed, the luminous intensity of the light source, and the like, they do not limit characteristics of the first luminous portion and the second luminous portion, and it is possible to obtain the above-described effect by turning on the first luminous portion and the second luminous portion in a switching manner though there may be differences in the degree of the effect. The same applies to descriptions of the following embodiments.

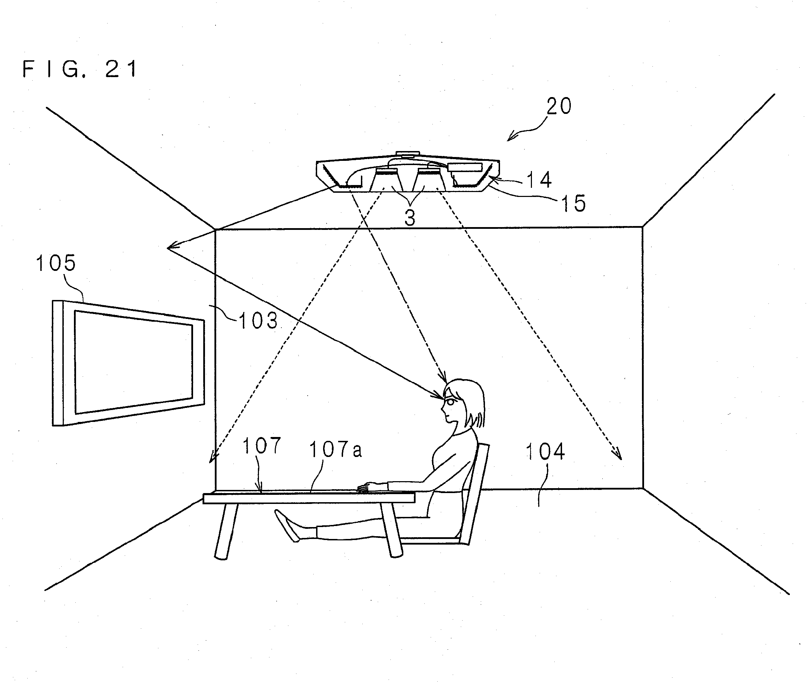

FIG. 21 is an explanatory view for explaining the amount of light entering the eyes of the user at still another scene in daily life. The user sits on a legless chair immediately below the lighting apparatus 20, and is watching television 105 hung on the opposing wall 103.

In this state, when only the first luminous portions 3, 3 ··· of the lighting apparatus 20 are turned on, since upper directions of more than 60° are generally out of the visual field, the light emitted from the first luminous portions 3, 3 ··· scarcely enters the eyes of the user. However, the light is emitted in a direction indicated by the broken line in the drawing, reflected by an upper surface 107a of a table 107, the floor surface 104, and the like, and part of the reflected light enters the eyes of the user.

On the other hand, when only the second luminous portion 14 is turned on, part of the light emitted from the second luminous portion 14 directly enters the eyes of the user as indicated by the one-dot chain line in the drawing, the rest of the light is reflected by the wall 103 and the like as indicated by the solid line in the drawing, and part of the reflected light further enters the eyes of the user. When compared with the cases in FIGS. 19 and 20, the user sits immediately below the lighting apparatus 20, and the amount of the light which is out of the visual field thereby increases so that the amount of the light directly entering the eyes of the user decreases. When the illuminance in the vicinity of the eyes of the user in a case where the second luminous portion 14 is turned on is 150 lx, the illuminance in the vicinity of the eyes of the user in a case where the first luminous portions 3, 3 ··· are turned on is about 50 Ix. Consequently, it is possible to change the amount of the light entering the eyes of the user by a factor of about three by turning on the first luminous portions 3, 3 ··· and the second luminous portion 14 in a witching manner.

In the lighting apparatus according to the present invention, in the normal usage mode described above, by turning on the first luminous portions 3, 3 ··· and the second luminous portion 14 in a switching manner, it is possible to change the amount of the light entering the eyes of the user by a factor of about three, and thereby perform adequate lighting in accordance with time or the lifestyle of the user. In addition to this, as described above, by changing the illuminance and the color temperature on the illuminated surface illuminated with the light from the first luminous portions 3, 3 ··· and the second luminous portion 14, it is possible to further change the amount of the light in the wavelength range inhibiting melatonin secretion which enters the eyes of the user, and thereby perform adequate lighting in accordance with time or the lifestyle of the user. Further, as described above, by turning on the plurality of blue light sources having different peak wavelengths in a switching manner to change emission spectrums of the first luminous portions 3, 3 ··· and the second luminous portion 14, it is possible to further change the amount of the light in the wavelength range inhibiting melatonin secretion which enters the eyes of the user, and thereby perform adequate lighting in accordance with time or the lifestyle of the user.

In each of the embodiments described above, as the plurality of luminous portions provided in the lighting apparatus, two types of luminous portions having different color temperatures, directivities, or light distribution characteristics are used. However, the number of types is not limited to two, and three or more types of luminous portions may also be used. In this case, since it is possible to control the light emitted from the lighting apparatus more finely, it is possible to control the light emitted in a predetermined direction (light entering the eyes of the user) more finely. Consequently, it is possible to further change the amount of the light in the wavelength range inhibiting melatonin secretion which enters the eyes of the user, and thereby perform adequate lighting in accordance with time or the lifestyle of the user.

### (Embodiment 6)

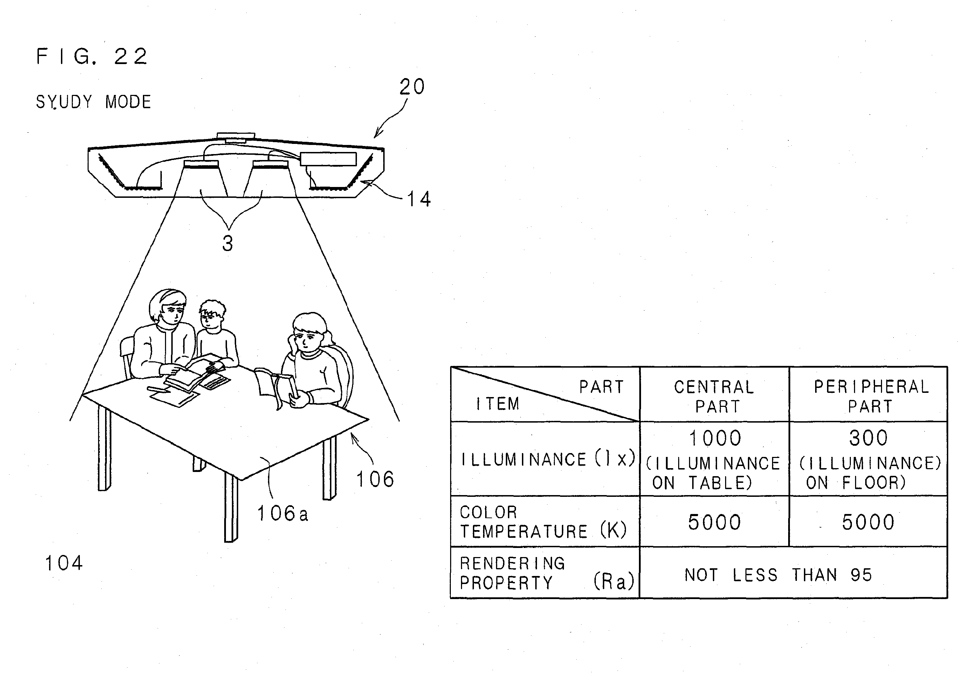

In each of the thus-configured lighting apparatuses, it is possible to freely set the color temperature and the color rendering property of the light emitted from the first luminous portion and the second luminous portion in accordance with the request and preference of the user while securing the above-described effect, and the present embodiment shows one example of such a setting. Hereinbelow, a description will be given by using the case where the lighting apparatus 20 according to Embodiment 4 is used as an example. FIG. 22 shows an example in which a lighting mode in the lighting apparatus 20 is set to a study mode. In recent years, the number of children who study in a living room is increasing, and there is desired a lighting apparatus capable of performing lighting suitable for studying as well as lighting suitable for the living room.

In the present embodiment, each of the first luminous portions 3, 3 ··· and the second luminous portion 14 is configured to be capable of having a color temperature of the luminous color of 5000 K, and an average color rendering index Ra of not less than 95. In addition, a configuration is adopted in which the first luminous portions 3, 3 ··· and the second luminous portion 14 are turned on with a high luminous intensity and a medium luminous intensity, respectively, whereby the illuminance on the upper surface 106a of the table 106 is set to 1000 lx, and the illuminance on the surrounding floor surface 104 is set to 300 lx. It is to be noted that the average color rendering index Ra is an index represented by values so as to be able to evaluate the color rendering property, and colors of an object look more natural as the average color rendering index Ra is higher.

By configuring each of the first luminous portions 3, 3 ··· and the second luminous portion 14 in this manner, the upper surface 106a of the table 106 is illuminated with light having a high color rending property at a sufficient illuminance of 1000 Ix so that it is possible to precisely reproduce colors in an illustrated book or the like, and conduct delicate work. In addition, the illuminance on the surrounding floor surface 104 is suppressed to be a medium illuminance (300 Ix) so that it is possible to increase concentration. By configuring the lighting apparatus 20 in this manner, it is possible to perform lighting optimum for study. It is to be noted that, by changing the respective luminous intensities of the first luminous portions 3, 3 ··· and the second luminous portion 14, it is possible to easily change the illuminance to that suitable for the living room.

Further, as described above, the light from the first luminous portions 3, 3 ··· scarcely enters the eyes of the user when the user is immediately below the lighting apparatus 20, and the luminous intensity of the second luminous portion 14 is suppressed to be a medium luminous intensity so that it is possible to suppress the amount of the light entering the eyes of the user, and to scarcely inhibit melatonin secretion.

### (Embodiment 7)

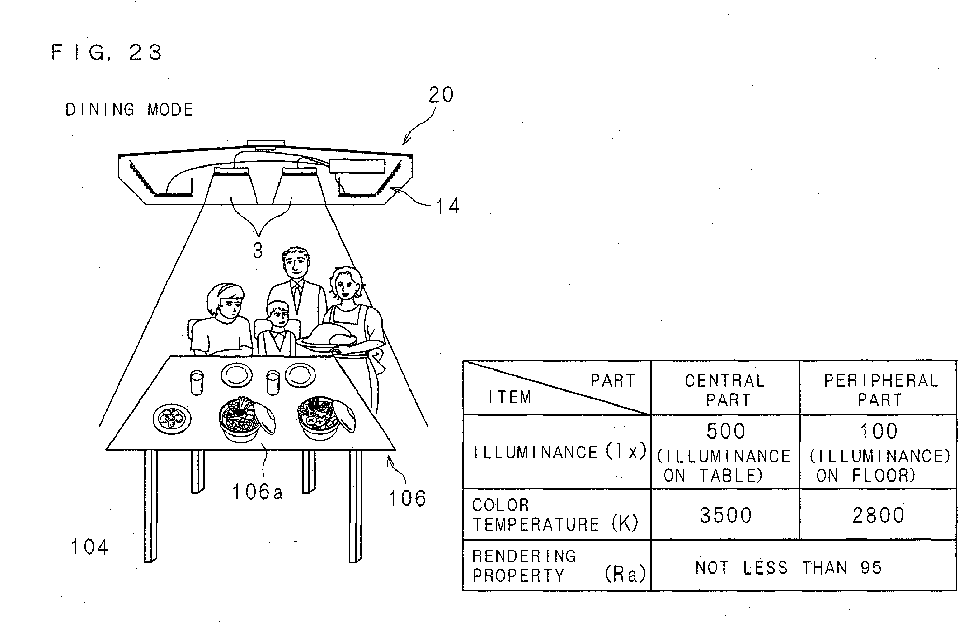

FIG. 23 shows an example in which the lighting mode of the lighting apparatus 20 is set to a dining mode. In the present embodiment, the first luminous portion 3, 3 ··· are configured to be capable of having a color temperature of the luminous color of 3500 K and an average color rendering index Ra of not less than 95, while the second luminous portion 14 is configured to be capable of having a color temperature of the luminous color of 2800 K and an average color rendering index Re of not less than 95. In addition, a configuration is adopted in which the first luminous portions 3, 3 ··· and the second luminous portion 14 are turned on with a medium luminous intensity and a low luminous intensity, respectively, whereby the illuminance on the upper surface 106a of the table 106 is set to 500 lx, and the illuminance on the floor surface 104 is set to 100 lx.

By configuring the first luminous portions 3, 3 ··· and the second luminous portion 104 in this manner, since the upper surface 106a of the table 106 is illuminated with light having a low color temperature of 3500 K and a high color rendering property of an average color rendering index Ra of not less than 95 so as to have a medium illuminance of 500 lx which is not too bright, it is possible to make food look delicious. Further, an LED is used as the light source so that an infrared ray is not emitted, and food is not warmed. Furthermore, since the surrounding floor surface 104 is illuminated at a low illuminance (100 lx) with light having a color temperature (2800 K) lower than that on the upper surface 106a of the table 106, it is possible to create an atmosphere as if dining at a restaurant, and make the user feel that the food is more delicious than it is. By configuring the lighting apparatus 20 in this manner, it is possible to perform lighting optimum for dining.

Moreover, as described above, the light from the luminous portions 3, 3 ··· scarcely enters the eyes of the user when the user is immediately below the lighting apparatus 20, and the luminous intensity of the second luminous portion 14 is suppressed to be low so that it is possible to suppress the amount of the light entering the eyes of the user, and to scarcely inhibit melatonin secretion.

### (Embodiment 8)

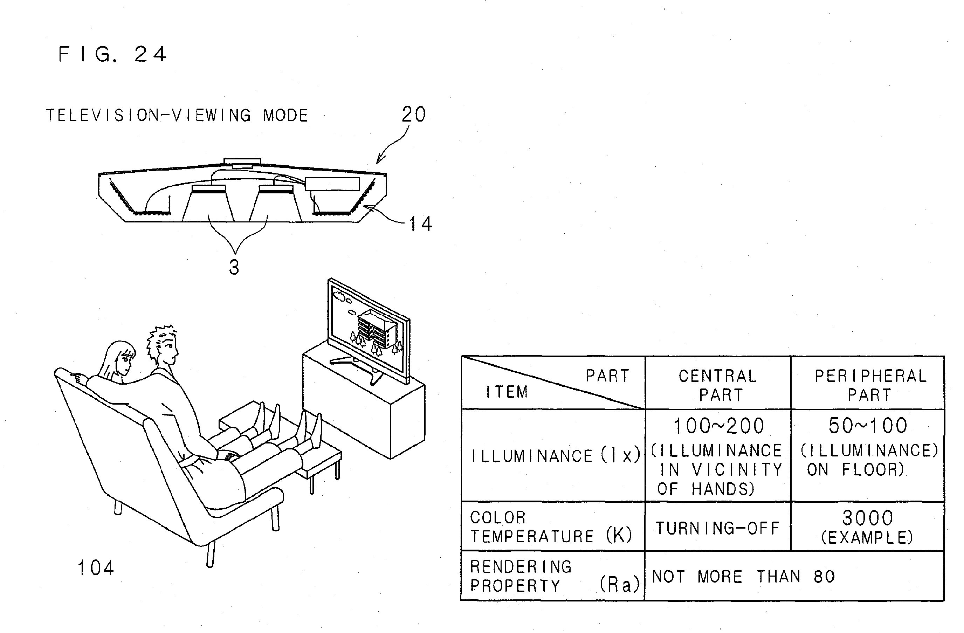

FIG. 24 shows an example in which the lighting mode of the lighting apparatus 20 is set to a television-viewing mode. In the present embodiment, a configuration is adopted in which the second luminous portion 14 is capable of having a color temperature of the luminous color of 3000 K and an average color rendering index Ra of not more than 80, and is turned on with a low luminous intensity, while the first luminous portions 3, 3 ··· are turned off, whereby the illuminance in the vicinity of hands of the user immediately below the lighting apparatus 20 is set to a range from 100 to 200 lx, and the illuminance on the surrounding floor surface 104 is set to a range from 50 to 100 lx.

By configuring the first luminous portions 3, 3 ··· and the second luminous portion 14 in this manner to illuminate the entire room at a low illuminance, it is possible to create space having a sense of realism. Since an area in the vicinity of hands of the user immediately below the lighting apparatus 20 is illuminated at an illuminance in the range from 100 to 200 lx, it is possible to obtain brightness sufficient to eat and drink, and operate a remote control unit. By configuring the lighting apparatus 20 in this manner, it is possible to perform lighting optimum for viewing a television. It is to be noted that it is desirable to configure the lighting apparatus 20 such that the luminous colors of the first luminous portions 3, 3 ··· and/or the second luminous portion 14 can be changed in order to enhance the sense of realism in accordance with images on a television screen.

Additionally, since the first luminous portions 3, 3 ··· are turned off, and the luminous intensity of the second luminous portion 14 is suppressed to be low, it is possible to suppress the amount of the light entering the eyes of the user, and to scarcely inhibit melatonin secretion.

### (Embodiment 9)

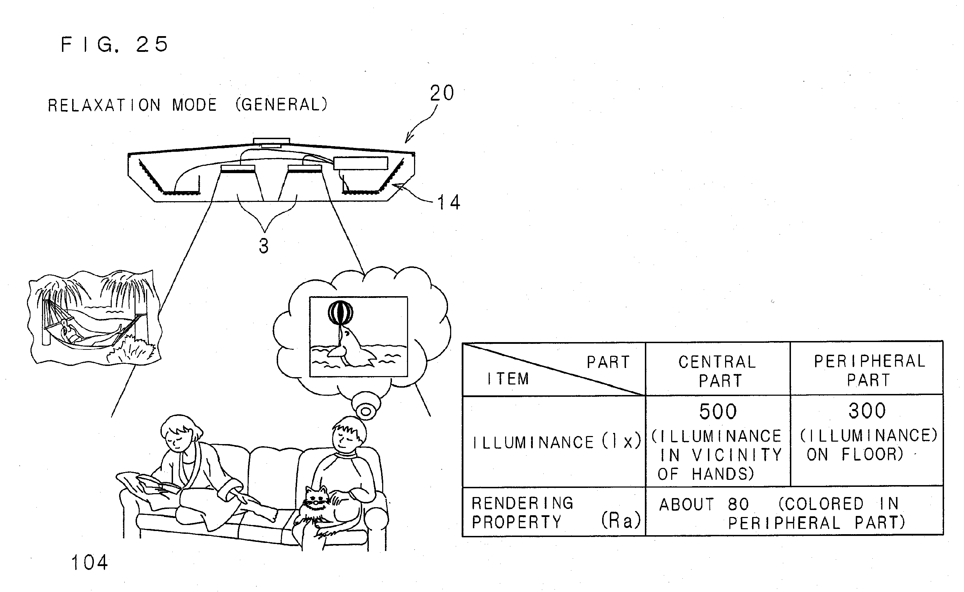

FIG. 25 shows an example in which the lighting mode of the lighting apparatus 20 is set to a relaxation mode. In the present embodiment, a configuration is adopted in which each of the first luminous portions 3, 3 ··· and the second luminous portion 14 is capable of having an average color rendering index Ra of about 80, and is turned on with a medium luminous intensity, whereby the illuminance in the vicinity of hands of the user immediately below the lighting apparatus 20 is set to 500 lx, and the illuminance on the surrounding floor surface 104 is set to 300 lx. The lighting apparatus 20 is configured such that the luminous color of the first luminous portions 3, 3 ··· is white, and the luminous color of the second luminous portion 14 is a color other than white, such as, e.g., blue. It is to be noted that the luminous color of the second luminous portion 14 is not limited to blue, and may be any color suitable for relaxation.

By configuring the first luminous portions 3, 3 ··· and the second luminous portion 14 in this manner to illuminate the entire room with light having a luminous color such as, e.g., a sky-blue color at appropriate brightness, it is possible to perform lighting optimum for relaxation.

It is to be noted that the lighting apparatus 20 may also be configured such that a plurality of lighting modes described in Embodiments 6 to 9 described above can be selected by the user in accordance with the use of a room where the lighting apparatus 20 is installed, a scene, or time. In this case, for example, a configuration is adopted in which the above-described infrared remote control unit 7 includes, in addition to the power switch for accepting the operation of turning on/off the lighting apparatus 20, a mode selection switch for accepting the operation of selecting one of the plurality of lighting modes, and an illuminance change switch for accepting the operation of increasing or decreasing the illuminance in the lighting mode selected by the mode selection switch, and the control portion 61 is capable of accepting the operations by the infrared remote control unit 7.

### (Embodiment 10)

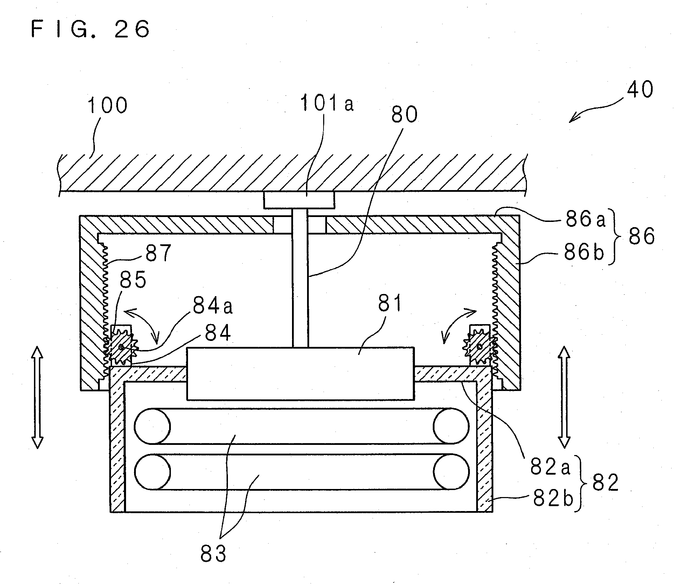

In the lighting apparatus according to each of Embodiments 1 to 9 described above, although the configuration is adopted in which a plurality of luminous portions having different light distribution characteristics are provided, and the amount of light emitted in a predetermined direction is changed by controlling the respective luminous intensities of the plurality of luminous portions, it is possible to obtain the similar effect by configuring the lighting apparatus such that the light distribution characteristics of the luminous portions are changed. FIG. 26 is a schematic view showing yet another configuration of a lighting apparatus.

In the drawing, the reference numeral 81 denotes an appliance main body of a lighting apparatus 40, and the appliance main body 81 is attached to a base plate 101a for attachment provided in the ceiling 100 via a hold portion 80 having a substantially cylindrical shape.

In the appliance main body 81, there is provided a cover 82 in a bottomed cylindrical shape. The cover 82 has a substantially disk-like plate portion 82a, and a peripheral wall portion 82b which extends from a peripheral edge of the plate portion 82a in one direction orthogonal to the plate portion 82a, and is provided with its side having the plate portion 82a being faced upward. The cover 82 is made of a milk-white translucent resin, and an example of the milk-white translucent resin includes a polycarbonate resin.

In addition, in the appliance main body 81, there are provided two annular fluorescent lamps 83 and 83 inside the cover 82 so as to be concentric with the cover 82. Each of the fluorescent lamps 83 and 83 is a light source which emits light with low directivity, and has light distribution characteristics represented by the view of the vertical light distribution curve shown in FIG. 18.

On an outer surface of the plate portion 82a of the cover 82, motors 84 and 84 are attached at the same intervals in a circumferential direction such that output shafts 84a and 84a of the motors 84 and 84 match the circumferential direction of the plate portion 82a. On the output shafts 84a and 84a of the motors 84 and 84, pinions 85 and 85 are coaxially provided.

On an upper side of the cover 82, there is provided an opaque cylindrical body 86 in a bottomed cylindrical shape. The cylindrical body 86 includes a substantially disk-like plate portion 86a having an outer diameter larger than that of the cover 82, and a peripheral wall portion 86b which extends from a peripheral edge of the plate portion 86a in one direction orthogonal to the plate portion 86a, and is provided with its side having the plate portion 86a being faced upward. On an inner peripheral surface of the peripheral wall portion 86b of the cylindrical body 86, racks 87 and 87 are formed at two positions disposed at the same intervals in a circumferential direction over a substantially entire length in an axial direction. The racks 87 and 87 are in gear with the pinions 85 and 85, respectively.

With the configuration described above, with the rotation of the motors 84 and 84, the pinions 85 and 85 provided on the output shafts 84a and 84a of the motors 84 and 84 are caused to rotate in a direction indicated by arrows and, in response to the rotation, the cylindrical body 86 formed with the racks 87 and 87 is caused to move vertically, as indicated by outline arrows. In the appliance main body 81, a control portion (not shown) is provided, and the control portion performs turning-on control of the fluorescent lamps 83 and 83, and drive control of the motors 84 and 84.

The lighting apparatus 40 according to the present embodiment is configured to drive the motors 84 and 84 according to time to cause the cylindrical body 86 to move in a vertical direction. Specifically, a configuration is adopted in which, in the time zone before bedtime, the cylindrical body 86 is caused to move in a downward direction to be at a substantially identical position with those of the fluorescent lamps 83 and 83 to shield light emitted in a substantially horizontal direction, while in the time zone in the morning and daytime, as shown in FIG. 26, the cylindrical body 86 is caused to move in an upward direction to be at the position higher than those of the fluorescent lamps 83 and 83 so as not to shield the light emitted in the substantially horizontal direction.

In the thus-configured lighting apparatus 40 according to the present embodiment, by changing relative vertical positions of the cylindrical body 86 and the fluorescent lamps 83 and 83 to regulate the amount of the light emitted in the substantially horizontal direction from the fluorescent lamps 83 and 83, it is possible to regulate the amount of the light in the wavelength range inhibiting melatonin secretion which enters the eyes of the user from the fluorescent lamps 83 and 83 to change the influence on the inhibition of melatonin secretion, and it is also possible to perform adequate lighting in accordance with time or the lifestyle of the user. It is to be noted that the illuminance immediately below the lighting apparatus 40 is held at a substantially constant value.

In the present embodiment, as light sources of the luminous portion, the fluorescent lamps 83 and 83 are used. However, the light source is not limited thereto, and various light sources such as an LED, an incandescent lamp, and the like may be used.

In addition, in the present embodiment, in order to convert the rotation of the motors 84 and 84 into the vertical movement of the cylindrical body 86, the pinions 85 and 85, and the racks 87 and 87 are used. However, it will be appreciated that components for the conversion are not limited thereto.

Further, in the present embodiment, a configuration is adopted in which the cylindrical body 86 is caused to move to change relative vertical positions of the cylindrical body 86 and the fluorescent lamps 83 and 83. However, a configuration may also be adopted in which the fluorescent lamps 83 and 83 are caused to move to change relative vertical positions of the cylindrical body 86 and the fluorescent lamps 83 and 83.

### (Embodiment 11)

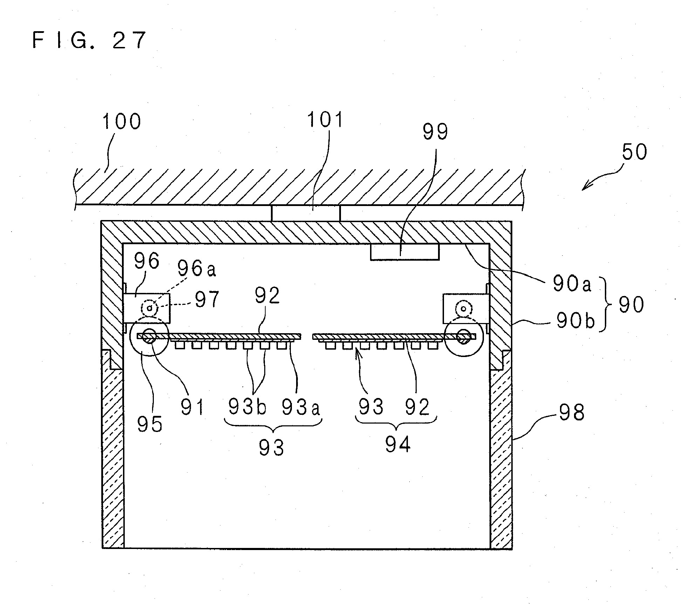

FIG. 27 is a schematic view showing still another configuration of a lighting apparatus. In the drawing, the reference numeral 90 denotes a frame which is a case with one opening surface, and the frame 90 includes a rectangular plate portion 90a, and rectangular four side walls 90b, 90b ··· which extend from peripheral edges of the plate portion 90a in one direction substantially orthogonal to the plate portion 90a.

In two opposing side walls 90b and 90b of the frame 90, shafts 91 and 91 are held rotatably. On the shafts 91 and 91, there are provided luminous portions 94 and 94 each including a rectangular support plate 92, and an LED module 93 attached to the support plate 92. In addition, at respective end parts of the shafts 91 and 91, first gears 95 and 95 are coaxially provided.

The LED module 93 includes an LED board 93a in a rectangular flat plate-like shape made of an epoxy resin, and a plurality of LEDs 93b, 93b ··· mounted on the LED board 93a, and has light distribution characteristics represented by the view of the vertical light distribution curve shown in FIG. 6.

To the other two opposing side walls 90b and 90b of the frame 90, motors 96 and 96 are attached with output shafts 96a and 96a thereof in parallel with the two opposing side walls 90b and 90b described above. On the respective output shafts 96a and 96a of the motors 96 and 96, second gears 97 and 97 are coaxially provided. The second gears 97 and 97 are in mesh with the first gears 95 and 95.

With the configuration described above, with the rotation of the motors 96 and 96, the second gears 97 and 97 provided on the output shafts 96a and 96a of the motors 96 and 96 are caused to rotate and, in response to the rotation, the first gears 95 and 95 are caused to rotate and, in turn, the luminous portions 94 and 94 are caused to move rotationally about the shafts 91 and 91 provided with the first gears 95 and 95 together with the shafts 91 and 91.

On the side with the opening of frame 90, a quadrilateral tubular panel 98 is externally fitted. The panel 98 is made of a milk-white translucent resin, and an example of the milk-white translucent resin includes a polycarbonate resin. On the plate portion 90a of the frame 90, there is provided a power source control circuit portion 99 including various circuit components such as a power transformer, a resistor, a capacitor, and the like. The control portion provided in the power source control circuit portion 99 performs turning-on control of the luminous portions 94 and 94, and drive control of the motors 96 and 96.

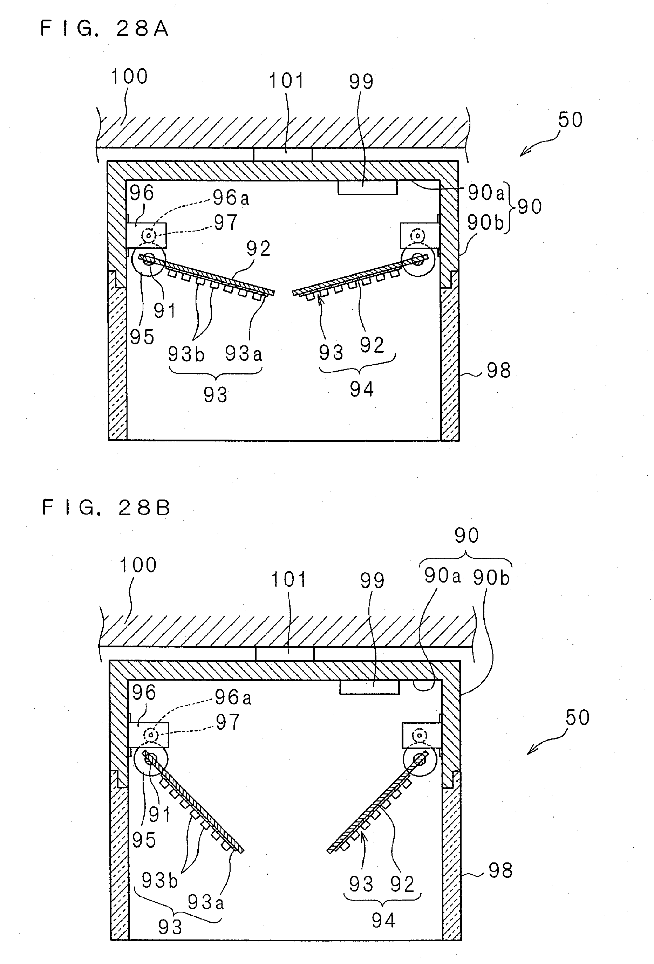

A lighting apparatus 50 according to the present embodiment drives the motors 96 and 96 in accordance with time to cause the luminous portions 94 and 94 to move rotationally. FIGS. 28A and 28B are explanatory views for explaining the operation of the lighting apparatus 50.

In the time zone before bedtime, as shown in FIG. 27, the luminous portions 94 and 94 are caused to move rotationally to set the support plates 92 to positions in parallel with the ceiling 100. As a result, most of light emitted by the luminous portions 94 and 94 is directed downward, and the remaining part of the light is directed in a substantially horizontal direction so that the amount of the light emitted in a substantially parallel direction which enters the eyes of the user in a normal usage mode decreases. Accordingly, it is possible to scarcely inhibit melatonin secretion to encourage smooth initiation of sleep.

In the morning, as shown in FIG. 28B, the luminous portions 94 and 94 are caused to move rotationally to set the support plates 92 and 92 at positions which form angles of about 45° with respect to the ceiling 100. As a result, most of the light emitted from the luminous portions 94 and 94 is directed in a substantially horizontal direction, and the remaining part of the light is directed in a downward direction so that the amount of the light emitted in a substantially horizontal direction which enters the eyes of the user in the normal usage mode increases. Accordingly, it is possible to inhibit melatonin secretion to encourage smooth awakening.

For, example, in the daytime, as shown in FIG. 28A, the luminous portions 94 and 94 are caused to move rotationally to set the support plates 92 and 92 to positions which form acute angles with respect to the ceiling 100. As a result, the light emitted from the luminous portions 94 and 94 is directed in a downward direction and a substantially horizontal direction so that it is possible to illuminate the entire room brightly.

In the thus-configured lighting apparatus 50 according to the present embodiment, by causing the luminous portions 94 and 94 to move rotationally to change the emission directions of the luminous portions 94 and 94, it is possible to regulate the amount of light in the wavelength range inhibiting melatonin secretion which enters the eyes of the user from the luminous portions 94 and 94 to change the influence on the inhibition of melatonin secretion, and also perform adequate lighting in accordance with time or the lifestyle of the user.

In the present embodiment, although the LED is used as the light source for each of the luminous portions 94 and 94, the light source is not limited thereto, and the light source may be any light source which emits light with directivity.

Although, in each of the embodiments described above, the description has been given of the lighting apparatus configured to be attached to the ceiling, the lighting apparatus is not limited thereto, and a table-lamp-type lighting apparatus placed on a floor can also be configured in the same manner. In this case, a predetermined direction in which the amount of light emitted from the luminous portion is changed is appropriately set in accordance with the position of the luminous portion in the lighting apparatus, the position where the lighting apparatus is installed, and the like.

In addition, in each of the embodiments described above, a configuration is adopted in which a duty ratio of a pulse signal generated in the control portion is changed to cause a switching element to open and close in accordance with the changed pulse signal, and the average current value to the LED is adjusted to change the luminous intensity of the luminous portion, whereby the control of the color temperature and the illuminance on the illuminated surface is implemented. However, the configuration is not limited thereto, and the control of the color temperature and the illuminance on the illuminated surface may be implemented by reducing the number of LEDs of the luminous portion which are turned on.

Further, it will be appreciated that the present invention may be embodied in various modified forms within the scope of the claims.

## Claims

1. A lighting apparatus comprising:
a plurality of luminous portions; and
control means for controlling turning-on of the plurality of luminous portions, wherein
the control means controls at least one of a luminous intensity and a color temperature of each of the plurality of luminous portions to regulate an amount of light in a wavelength range inhibiting melatonin secretion which is emitted in a predetermined direction.

2. The lighting apparatus of claim 1, wherein
each of the plurality of luminous portions has a different directivity, and at least one of the plurality of luminous portions has a light source, and a shield which shields light emitted in a predetermined direction from the light source.

3. The lighting apparatus of claim 1 or 2, which is attachable to a ceiling, and changes an amount of light emitted in a substantially horizontal direction from the luminous portion.

4. The lighting apparatus of any one of claims 1 to 3, wherein the control means controls the luminous intensity of the luminous portion to change an illuminance on an illuminated surface illuminated with light from the luminous portion.

5. The lighting apparatus of any one of claims 1 to 4, wherein the luminous portion includes a plurality of light sources having different luminous colors, and the control means controls the luminous intensity of each of the plurality of light sources to change the color temperature on an illuminated surface illuminated with light from the plurality of light sources.

6. The lighting apparatus of any one of claims 1 to 5, wherein the luminous portion has a plurality of light sources including a red light source, a green light source, and a plurality of blue light sources having different peak wavelengths, and the control means turns on the plurality of blue light sources in a switching manner.

7. The lighting apparatus of any one of claims 1 to 6, wherein the control means includes a clock portion, and controls the luminous intensity of the luminous portion in accordance with time of the clock portion.

8. The lighting apparatus of any one of claims 1 to 7, comprising:
an illuminance sensor for detecting the illuminance, wherein
the control means controls the luminous intensity of the luminous portion in accordance with the illuminance detected by the illuminance sensor.
